# EUROPEAN PATENT APPLICATION

(11) **EP 4 629 260 A1**
(43) Date of publication of application: **08.10.2025**
(21) Application number: 24760479.6
(22) Date of filing: 15.01.2024
(51) Int. Cl.: G16H 20/30, G16H 10/60, G06N 3/092, G06N 20/00, A63B 24/00, G06N 3/08

(54) **METHOD AND SERVER FOR PROVIDING PERSONALIZED RECOMMENDATION ON BASIS OF REINFORCEMENT LEARNING**

(30) Priority: 22.02.2023 KR 20230023641; 06.09.2023 KR 20230118553
(71) Applicant: Samsung Electronics Co., Ltd., Suwon-si, Gyeonggi-do 16677 (KR)
(72) Inventor: GERACI, James Russell, Suwon-si, Gyeonggi-do, 16677 (KR); SHI, Bichen, Suwon-si, Gyeonggi-do, 16677 (KR); O'REILLY-MORGAN, Diarmuid, Suwon-si, Gyeonggi-do, 16677 (KR); LAWLOR, Aonghus, Suwon-si, Gyeonggi-do, 16677 (KR); SMYTH, Barry, Suwon-si, Gyeonggi-do, 16677 (KR); TRAGOS, Ilias, Suwon-si, Gyeonggi-do, 16677 (KR); HURLEY, Neil, Suwon-si, Gyeonggi-do, 16677 (KR)
(74) Representative: Gulde & Partner
(86) International application number: PCT/KR2024/000714
(87) International publication number: WO 2024/177287

(57) **Abstract**

A method of providing a personalized recommendation based on reinforcement learning is provided. The method may include: obtaining user data; generating a simulated user representing a virtual user corresponding to an actual user who receives a recommendation, based on the user data; determining an action based on the simulated user state, wherein the action determines a recommendation element to be included in a recommendation session to be provided to the user; updating the state of the simulated user; identifying an updated state of the simulated user and a reward for the recommendation element output from the simulated user; generating a personalized recommendation session by repeatedly determining the recommendation element based on the updated state of the simulated user and the reward; and outputting the personalized recommendation session.

## Description

### Technical Field

The present disclosure relates to a method, a server, and an electronic device for providing personalized recommendations to a user based on reinforcement learning using a user simulator.

### Background Art

Reinforcement learning is a field of machine learning in which agents learn actions that maximize rewards while interacting with their environments. When the reinforcement learning is applied to a recommendation system, an agent of a reinforcement learning model can learn a recommendation strategy that maximizes rewards obtained in a process of taking actions while interacting with a user. Meanwhile, a problem of lack of data is one of important considerations in reinforcement learning, as in general machine learning techniques. In some reinforcement learning algorithms, techniques such as transfer learning or pre-training are used to solve the problem of lack of data. In consideration of the above, the present disclosure proposes a technique for precisely training and utilizing reinforcement learning while overcoming the problem of lack of data in reinforcement learning.

### Disclosure of Invention

### Solution to Problem

According to an aspect of the present disclosure, a method, performed by a server, of providing a personalized recommendation session based on reinforcement learning may be provided. The method may include obtaining user data. The method may include generating a simulated user representing a virtual user corresponding to an actual user who receives a recommendation, based on the user data. The method may include determining an action based on the simulated user state, wherein the action determines a recommendation element to be included in a recommendation session to be provided to the user. The method may include updating the state of the simulated user. The method may include identifying an updated state of the simulated user and a reward for the recommendation element output from the simulated user. The method may include generating a personalized recommendation session by repeatedly determining the recommendation element based on the updated state of the simulated user and the reward. The method may include outputting the personalized recommendation session.

According to an aspect of the present disclosure, a server for providing a personalized recommendation session based on reinforcement learning may be provided. The server may include: a communication interface; memory storing at least one instruction; and at least one processor configured to execute the at least one instruction stored in the memory. The at least one processor may be configured to execute the at least one instruction to obtain user data. The at least one processor may be configured to execute the at least one instruction to generate a simulated user representing a virtual user corresponding to an actual user who receives a recommendation, based on the user data. The at least one processor may be configured to execute the at least one instruction to determine an action based on the simulated user state, wherein the action determines a recommendation element to be included in a recommendation session to be provided to the user. The at least one processor may be configured to execute the at least one instruction to update a state of the simulated user. The at least one processor may be configured to execute the at least one instruction to identify an updated state of the simulated user and a reward for the recommendation element output from the simulated user. The at least one processor may be configured to execute the at least one instruction to generate a personalized recommendation session by repeatedly determining the recommendation element based on the updated state of the simulated user and the reward. The at least one processor may be configured to execute the at least one instruction to output the personalized recommendation session.

According to an aspect of the present disclosure, a computer-readable recording medium storing a program for executing any one of the methods which have been described above or will be described below to provide a personalized recommendation session based on reinforcement learning, the methods being performed by a server, may be provided.

### Brief Description of Drawings

FIG. 1 is a diagram schematically illustrating a server providing a personalized recommendation session based on reinforcement learning, according to an embodiment of the present disclosure.
FIG. 2 is a flowchart for describing an operation, performed by a server according to an embodiment of the present disclosure, of providing a personalized recommendation session.
FIG. 3 is a diagram illustrating an overall structure of a reinforcement learning model that provides a workout recommendation, according to an embodiment of the present disclosure.
FIG. 4 is a diagram for describing an operation, performed by a sever according to an embodiment of the present disclosure, of generating a simulated user.
FIG. 5 is a diagram for describing an operation, performed by a sever according to an embodiment of the present disclosure, of generating a personalized recommendation session.
FIG. 6 is a diagram for describing an operation, performed by a server 2000 according to an embodiment of the present disclosure, of re-training a simulated user.
FIG. 7A is a diagram for describing an operation of a user simulator according to an embodiment of the present disclosure.
FIG. 7B is a diagram for describing an operation of a user simulator according to an embodiment of the present disclosure.
FIG. 8 is a diagram for describing an operation, performed by a server according to an embodiment of the present disclosure, of training a user simulator.
FIG. 9 is a diagram for describing an operation, performed by a server according to an embodiment of the present disclosure, of generating data for training a reinforcement learning model.
FIG. 10A is a diagram for describing an operation, performed by a server according to an embodiment of the present disclosure, of obtaining user data to provide a personalized recommendation providing service.
FIG. 10B is a diagram for describing an operation, performed by a server according to an embodiment of the present disclosure, of obtaining user data to provide a personalized recommendation providing service.
FIG. 11A is a diagram for describing an operation, performed by a server according to an embodiment of the present disclosure, of providing a personalized recommendation providing service.
FIG. 11B is a diagram for describing an operation, performed by a server according to an embodiment of the present disclosure, of obtaining feedback on a personalized recommendation providing service.
FIG. 11C is a diagram for describing an operation, performed by a server according to an embodiment of the present disclosure, of obtaining feedback on a personalized recommendation providing service.
FIG. 11D is a diagram for describing an operation, performed by a server according to an embodiment of the present disclosure, of obtaining feedback on a personalized recommendation providing service.
FIG. 12 is a diagram for describing an operation, performed by a server according to an embodiment of the present disclosure, of additionally providing information related to a provided recommendation session.
FIG. 13 is a diagram for describing an example of a server, according to an embodiment of the present disclosure, providing a personalized recommendation session.
FIG. 14 is a diagram for describing an example of a server, according to an embodiment of the present disclosure, providing a personalized recommendation session.
FIG. 15 is a block diagram showing a configuration of a server according to an embodiment of the present disclosure.
FIG. 16 is a block diagram showing a configuration of an electronic device according to an embodiment of the present disclosure.

### Mode for the Invention

Terms used in this specification will be briefly described, and the present disclosure will be described in detail. In the present disclosure, the expression "at least one of a, b or c" indicates "a", "b", "c", "a and b", "a and c", "b and c", "all of a, b, and c", or variations thereof.

Although general terms being currently widely used were selected as terminology used in the present disclosure while considering the functions of the present disclosure, they may vary according to intentions of one of ordinary skill in the art, judicial precedents, the advent of new technologies, and the like. Also, terms arbitrarily selected by the applicant of the present disclosure may also be used in a specific case. In this case, their meanings will be described in detail in the detailed description of the present disclosure. Hence, the terms used in the present disclosure must be defined based on the meanings of the terms and the contents of the entire specification, not by simply stating the terms themselves.

The singular forms "a," "an," and "the" include plural referents unless the context clearly dictates otherwise. All terms used herein, including technical or scientific terms, have the same meaning as commonly understood by one of ordinary skill in the technical art written in the present specification. Also, in the present specification, although the terms including ordinal numbers, such as "first", "second", etc., may be used herein to describe various components, the components should not be limited by these terms. These terms are only used to distinguish one component from another.

In the entire specification, it will be understood that when a certain part "includes" a certain component, the part does not exclude another component but can further include another component, unless the context clearly dictates otherwise. In addition, the terms "portion", "part", "module", etc. used in this specification refer to a unit for processing at least one function or operation, which is implemented as hardware, software, or a combination of hardware and software.

Hereinafter, embodiments of the present disclosure will be described in detail with reference to the accompanying drawings so that the present disclosure may be readily implemented by one of ordinary skill in the technical field to which the present disclosure pertains. However, the present disclosure is not limited to these embodiments and may be embodied in various other forms. In the drawings, parts irrelevant to the description are omitted to clearly describe the present disclosure, and like reference numerals refer to like elements throughout the specification.

Hereinafter, the present disclosure will be described in detail with reference to the accompanying drawings.

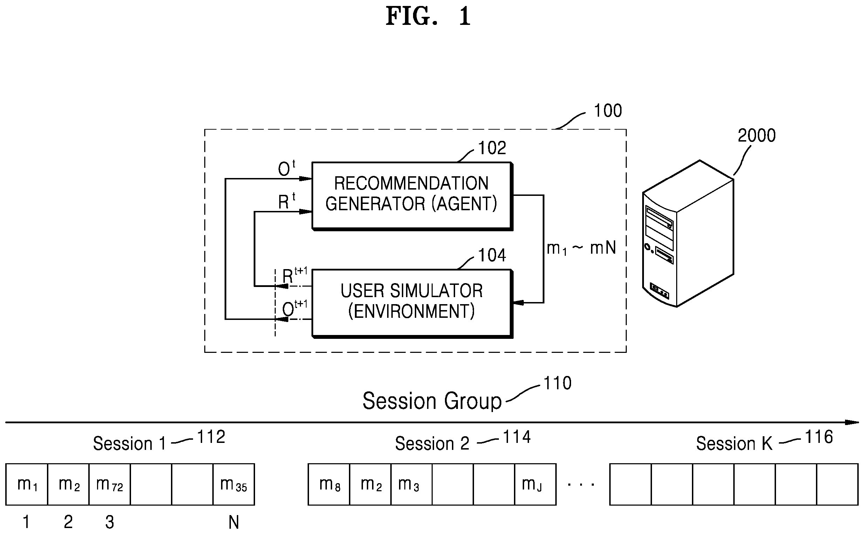

FIG. 1 is a diagram schematically illustrating a server providing a personalized recommendation session based on reinforcement learning, according to an embodiment of the present disclosure.

Referring to FIG. 1, a server 2000 according to an embodiment may provide a personalized recommendation to a user by using a reinforcement learning model 100.

In a general reinforcement learning system, an agent may determine an action and perform the action while interacting with an environment. An agent of reinforcement learning may be trained to select an action to maximize a reward by recognizing a current state and observing an environment.

In an embodiment, the reinforcement learning model 100 may include a recommendation generator 102 and a user simulator 104. In the reinforcement learning model 100 of the present disclosure, the recommendation generator 102 may perform, as an agent, an action that determines a recommendation element for a user. Also, an actual user (or a recommendation providing application) that receives a recommendation element may correspond to an environment and interact with the agent.

In an embodiment, the reinforcement learning model 100 may generate a simulated user by using the user simulator 104. The simulated user may refer to synthetic data that represents a virtual user corresponding to the actual user. Also, the user simulator 104 may be generative artificial intelligence. The user simulator 104 may have been trained in advance, and the user simulator 104 may have been trained for a simulated user to provide "simulated feedback" as virtual feedback that is similar to that from the actual user. The embodiment of the present disclosure may provide an advantage of training the reinforcement learning model 100 only with a small amount of actual user data by training the reinforcement learning model 100 using a simulated user.

In an embodiment, the recommendation generator 102 may perform an action of determining any recommendation element from among candidate recommendation elements configured with recommendation elements m1 to mN, the user simulator 104 may generate a simulated user that simulates the actual user, and the simulated user may interact with the recommendation generator 102. The recommendation generator 102 may observe, for example, a state of the simulated user and determine any one of the recommendation elements m1 to mN based on the state of the simulated user. The simulated user generated by the user simulator 104 may output a reward for the recommendation element received from the recommendation generator 102, and the reinforcement learning model 100 may be trained to optimize a process of determining an action of selecting a recommendation element to maximize the reward. In the present disclosure, the reinforcement model 100 may be a model trained in advance to provide a recommendation to a user, and the reinforcement model 100 may be referred to as a trained reinforcement learning model or a distributed reinforcement learning model. Also, the reinforcement model 100 may be referred to simply as a reinforcement learning model by omitting modifiers. Meanwhile, the reinforcement learning model 100 may be retrained based on data collected while the user uses the reinforcement learning model 100.

In an embodiment, the server 2000 may provide at least one recommendation session to the user by using the reinforcement learning model 100. For example, the server 2000 may generate a total of K recommendation sessions, including session 1 (112), session 2 (114), ..., session K (116). In the present disclosure, a plurality of recommendation sessions provided by the server 2000 may be referred to as a recommendation session group 110.

In an embodiment, the server 2000 may provide the user with the recommendation session group 110 for various categories. For example, the server 2000 may provide the user with the recommendation session group 110 corresponding to various categories, such as workout recommendations, diet recommendations, and media content recommendations.

More specifically, for example, the server 2000 may generate a workout recommendation session group including a plurality of workout recommendation sessions by repeatedly generating workout recommendation sessions. In this case, the workout recommendation session group may correspond to an entire workout, the entire workout may be configured with the plurality of workout recommendation sessions, and each of the plurality of workout recommendation sessions may be configured with movement recommendation elements. Here, the movement may indicate a specific motion (for example, squats, etc.) of a workout. The server 2000 may provide a workout recommendation session to the user, and the user may perform one workout session by following N movements received from the server 2000.

Hereinafter, an example in which the server 2000 according to the present disclosure provides a personalized recommendation session to a user will be assumed to be a workout recommendation scenario, unless otherwise stated. However, this is only an example for convenience of description, and a personalized recommendation provided by the server 2000 according to the present disclosure may be applied in the same/similar manner to other recommendation categories than workout. That is, the present disclosure may be applied to various fields in which a personalized recommendation session may be provided to a user, and the present disclosure may provide a personalized recommendation session to a user by using a user simulated based on reinforcement learning.

An operation of, performed by the server 2000, providing a personalized recommendation to a user through reinforcement learning using a simulated user will be described in more detail with reference to the accompanying drawings.

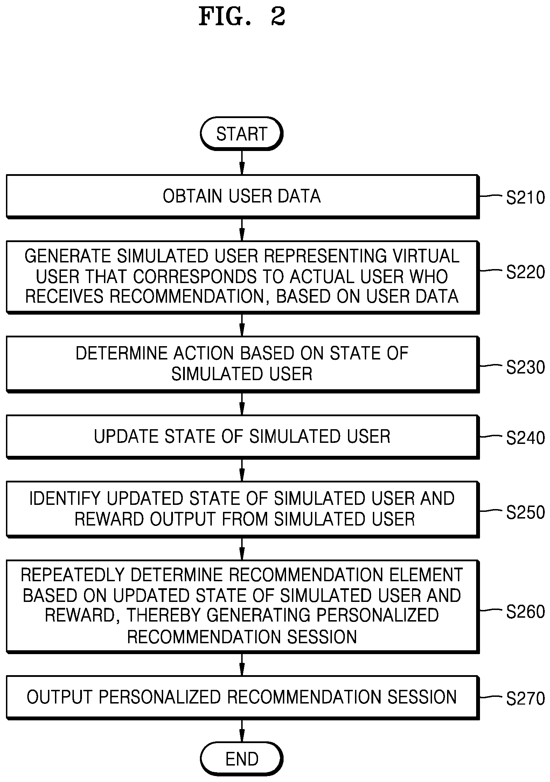

FIG. 2 is a flowchart for describing an operation of, performed by a server according to an embodiment of the present disclosure, providing a personalized recommendation session.

Overall operations of the server 2000 according to the present disclosure will be described with reference to FIG. 2. Also, details of the operations of the server 2000 will be described with reference to the following drawings.

In operation S210, the server 2000 may obtain user data. The user data may have been stored in the server 2000 or may be received from the user's electronic device (a mobile phone, etc.). The server 2000 may receive a recommendation service providing request from the user.

In an embodiment, the user data may have been input by the user who will receive a recommendation session. The user data may include a basic description for identifying the user's tendency. For example, the user data may include personal information about the user, such as gender and age. Also, for example, the user data may include user input information related to a recommendation category. More specifically, in a workout recommendation scenario, the user data may include a workout style, a workout duration, focused muscles, workout proficiency, etc. Also, in a diet recommendation scenario for weight loss, the user data may include information about food allergies, a region, a budget, weight, a blood sugar level, a dining place, accessibility to cooking, etc. Also, in a long-term content recommendation scenario, the user data may include genre preferences, language preferences, a region, interests, a hobby, a marital status, presence of children, etc.

The server 2000 according to an embodiment may obtain user data corresponding to a recommendation category in order to provide a recommendation of a specific category to the user. In this case, the user data may be input by the user. For example, the server 2000 may obtain user data corresponding to a predetermined recommendation category. Also, the server 2000 may obtain user data and identify a recommendation category based on data elements included in the user data. The server 2000 may perform the following operations to generate a recommendation session configured with a plurality of recommendation elements, based on the user data.

In operation S220, the server 2000 may generate a simulated user representing a virtual user that corresponds to an actual user who will receive a recommendation, based on the user data.

In an embodiment, the server 2000 may generate the simulated user by using a user simulator which is pretrained generative artificial intelligence. In the reinforcement learning system according to the present disclosure, the actual user (or a recommendation providing application) who will receive the recommendation may correspond to an environment. Also, a generator which determines a recommendation element and provides the recommendation element may correspond to an agent. As a detailed example, the simulated user may be obtained by simulating an actual user who will receive a workout recommendation. The simulated user may be used to track the actual user's internal state and manage a history of recommendations provided to the actual user. Also, the simulated user may generate simulated feedback that is used to train a reinforcement learning model. In this case, the user simulator may have been trained to generate simulated feedback that is similar to that from actual users based on feedback data of the actual users.

In operation S230, the server 2000 may determine an action based on a state of the simulated user. Here, the action may be to determine a recommendation element from among recommendation element candidates, and the action may be performed by a generator which is an agent.

In an embodiment, to provide a workout recommendation session, the server 2000 may determine movements which are movement recommendation elements related to a workout based on the state of the simulated user. Movement recommendation elements included in a workout recommendation session may refer to basic workout motions included in one workout session. For example, the movement recommendation elements may be, but are not limited to, squats, burpees, etc.

A user's state may be divided into various segments (s = Concat(s1, s2, s3, ..., sn)). The segments of the user's state may include at least one of, for example, a user description d, user preferences p, a user internal state i, or a recommendation history h.

In an example of a workout recommendation scenario, the user description may include personal information about the user and user input information (for example, a workout style, a workout duration, focused muscles, workout proficiency, etc.) related to a recommendation category.

Also, the user preferences may include movement preferences, movement difficulty level preferences, variety preferences, feedback preferences, etc.

Also, the user internal state may include elements that are affected when the user receives a recommendation. For example, fitness improvement, a real-time heart rate, and muscle fatigue, etc. may be included in the user internal state.

Also, the recommendation history may include a history about recommendation elements recommended to the user and feedback on the recommendation elements. For example, movements which are recommendation elements included in a workout and feedback on the movements may be included in the recommendation history.

In operation S240, the server 2000 may update the state of the simulated user. This may be expressed as a natural update of a state because the state is transitioned according to an action by a state transition function T(s, a, s'). The simulated user may be a virtual environment (virtual user) that simulates an actual environment (user). The state of the simulated user may be updated based on the state transition function, and the server 2000 may manage an interaction between the simulated user which is an environment and the generator which is an agent. The state transition function may be a function that defines how a transition to a next state s' occurs according to a current state s and an action a selected by the agent while the agent interacts with the environment. That is, the state transition function may include a probability that a current state is transitioned to a next state according to a specific action, and may be modeled in a process of training the reinforcement learning model.

In an embodiment, according to the server 2000 selecting a movement among movement candidates as a recommendation element, the user description d, the user preferences p, the user internal state i, the recommendation history h, etc. included in the state of the simulated user may be updated.

In operation S250, the server 2000 may identify an updated state of the simulated user and a reward output from the simulated user.

A reward function R(s, a, s') may be a function that defines numerical values provided when a state s is transitioned to a next state s'. In the present disclosure, a reward may be defined to correspond to an action in which an agent observes a current state and determines a recommendation element. That is, in the present disclosure, because a reward is independent of a state to which an environment is transitioned, a reward function may be defined as R(s, a) which is not affected by a next state s'. An agent may learn a policy of determining an action based on a reward while interacting with an environment, and therefore, the agent will find better optimal actions as requests for recommendation sessions are repeated in the future.

Rewards may be designed to include negative rewards and positive rewards. For example, the negative rewards may include a case in which a user does not like a recommendation element, and the positive rewards may include a case in which a user likes a recommendation element.

In an embodiment, the server 2000 may provide a movement which is a recommendation element included in a recommendation session in order to provide a workout recommendation session, and identify a negative reward or a positive reward on the recommendation element.

For example, the negative rewards may include a case in which a user does not like a recommended movement, a case in which a user determines that a recommended movement is too easy or difficult, a case in which a recommended movement is excessively repeated, a case in which a recommended movement does not meet a user's workout style, etc. However, the negative rewards are not limited to the above-described examples.

For example, the positive rewards may include a case in which a user likes a recommended movement, a case in which a recommended movement meets a user's preferred workout difficulty level, a case in which a recommended movement improves variety of movements in a workout recommendation session, a case in which a recommended movement corresponds to focused muscles of user data, etc. However, the positive rewards are not limited to the above-described examples.

In operation S260, the server 2000 may repeatedly determine a recommendation element based on the updated state of the simulated user and the reward, thereby generating a personalized recommendation session.

In reinforcement learning, an agent may repeat an operation of determining an action while interacting with an environment until an episode (a sequence of actions) of the reinforcement learning terminates.

In an embodiment, the server 2000 may repeatedly determine a recommendation element based on a termination condition of an episode. For example, the server 2000 may determine a recommendation element N times to generate a recommendation session including N recommendation elements. More specifically, the server 2000 may generate a workout recommendation session including N movement recommendation elements.

In an embodiment, the server 2000 may repeatedly generate a recommendation session to generate a recommendation session group including a plurality of recommendation sessions. For example, the server 2000 may repeatedly generate a workout recommendation session to generate a workout recommendation session group including a plurality of workout recommendation sessions. In this case, the workout recommendation session group may correspond to an entire workout, the entire workout may be configured to the plurality of workout recommendation sessions, and each of the plurality of workout recommendation sessions may be configured with movement recommendation elements.

In an embodiment, the server 2000 may provide a recommendation session to the user and obtain feedback from the user. The server 2000 may retrain the simulated user based on the feedback from the user. The server 2000 may retrain the simulated user whenever a recommendation session is generated to cause the simulated user to precisely simulate the actual user's behavior, thereby providing the user with a gradually personalized recommendation session.

In operation S270, the server 2000 may output the personalized recommendation session. The server 2000 may transmit the personalized recommendation session to the user's electronic device (for example, a mobile phone).

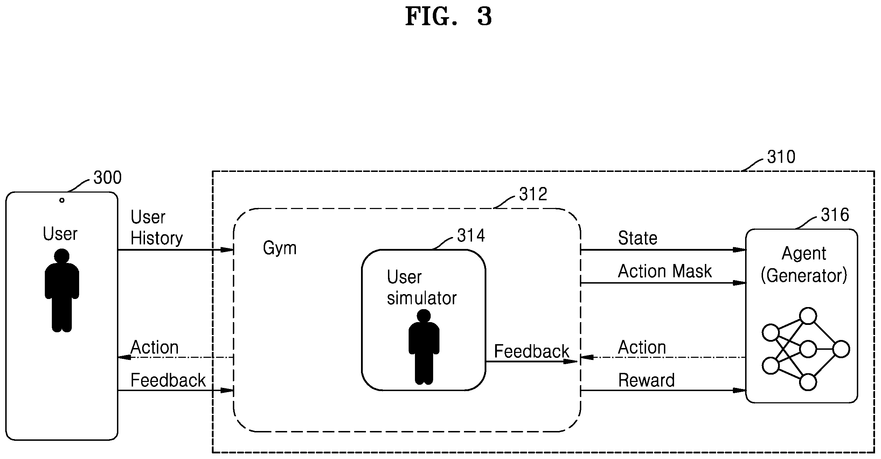

FIG. 3 is a diagram illustrating an overall structure of a reinforcement learning model that provides a workout recommendation, according to an embodiment of the present disclosure.

Referring to FIG. 3, an actual user 300 who receives a workout recommendation may interact with a reinforcement learning model 310 by using an application (for example, a mobile application, a web application, etc.). The user may receive workout recommendation elements (actions) from the reinforcement learning model 310 and provide feedback on the recommendation elements and a workout history (a user history) to the reinforcement learning model 310.

In an embodiment, the reinforcement learning model 310 that provides a workout recommendation may include a virtual environment (also referred to as RL Gym) 312 constructed to represent an environment of the reinforcement learning model 310. Also, the reinforcement learning model 310 may include an agent (also referred to as a generator) 316 that interacts with the virtual environment 312 or the user 300 who is an actual environment.

In an embodiment, the virtual environment 312 may include a user simulator 314 which is synthetic data and functions to generate interactions. The user simulator 314 may generate a simulated user which is synthetic data, generate simulated feedback, and provide the simulated feedback to the agent 316. That is, the user simulator 314 may generate synthetic data to train the agent 316 only with limited actual data.

In an embodiment, the reinforcement learning model 310 may use a Markov Decision Process (MDP) being a mathematical model that probabilistically models a sequential decisionmaking problem. The agent 316 of the reinforcement learning model 310 may select a specific action in each state, and when an action is selected in a current state, a next state may be determined by a state transition function. A reward that is obtained in each state may be determined by an action, and the reward may be defined by reflecting an ultimate goal (for example, providing a personalized workout session recommendation) of the agent. The virtual environment 312 may transmit a current state of the environment to the agent 316, receive an action from the agent 316, and then provide a reward and a new state/observation to the agent 316. For example, the virtual environment 312 may transmit a current state of the simulated user to the agent 316, receive a workout recommendation element from the agent 316, then update the state of the simulated user, and provide a reward and a new state/observation to the agent 316.

Hereinafter, a state, an action, a reward, feedback, etc. will be described in detail to describe an operation of the reinforcement learning model 310 according to the present disclosure.

In an embodiment, the virtual environment 312 may transfer a state to the agent 316. That is, the agent 316 may observe the virtual environment 312 and identify a state. The state may include all information required to determine a reward at a given time and a transition to a subsequent state. The state may be divided into various segments (s= Concat(s1, s2, s3, ..., sn)).

In a workout recommendation scenario, for example, some segments of a state may include, but are not limited to, at least one of a user description d, user preferences p, a user internal state i, or a recommendation history h.

The user description included in the state may include, but is not limited to, personal information (for example, age, gender, etc.) related to the user and user input information (for example, a workout goal, a workout style, a workout duration, focused muscles, workout proficiency, a fitness level, etc.) related to a workout which is a recommendation category.

The user preferences included in the state may include, but is not limited to, movement preferences, movement difficulty level preferences, variety preferences, heart rate preferences, feedback preferences (a probability of providing a specific type of feedback), etc.

The user internal state included in the state may include elements that are affected when the user receives a recommendation. For example, the user internal state may include, but is not limited to, fitness improvement, a real-time heart rate, and muscle fatigue, etc.

The recommendation history included in the state may include a history about recommendation elements recommended to the user and feedback on the recommendation elements. For example, movements which are recommendation elements included in a workout, feedback on the movements, etc. may be included in the recommendation history.

In an embodiment, segments included in a state may be classified into "observable " or "unobservable (or partially observable)". The observable state may be a state for which an agent may directly observe an exact state of an environment. For example, a user description (a workout goal, an initial fitness level, a workout style, etc.) and a recommendation history may be classified into observable. The unobservable or partially observable state may be a state for which an agent obtains information about an exact state of an environment through indirect observation or inference because the agent is incapable of directly observing the exact state of the environment. For example, user preferences and a user internal state may be classified into unobservable or partially observable. In this case, the MDP of the reinforcement learning model 310 may be a Partially observable Markov Decision Process (POMDP).

In an embodiment, the state transition function may define how a current state is transitioned to a next state when the agent 316 takes a specific action in a specific state. That is, the state transition function T(s, a, s') may be a function that defines how a current state s is transitioned to a next state s' according to an action a selected by the agent 316 while the agent 316 interacts with the virtual environment 312 or the actual user 300. In an embodiment, segments included in a state may be classified into "static" or "dynamic".

For example, a user description and user preferences included in a state may be classified into static. In the state transition function, a static segment may be defined to be maintained as it is even after a transition to a next state occurs.

For example, a user internal state and a recommendation history included in a state may be classified into dynamic. In the state transition function, a dynamic segment may be defined to change when a transition to a next state occurs. In this case, the dynamic segment may be classified into "deterministic (p=1)" or "non-deterministic (p≠1)".

For example, a recommendation history, which is a dynamic segment, may be classified into deterministic because the recommendation history results from newly adding a recommendation element determined by a current action to an end of a list of recommendation sessions. Also, user feedback, such as like/dislike for a recommended element, may be classified into non-deterministic because the user feedback is defined by a probability. Likewise, a state transition of a user internal state may also be classified into non-deterministic. Because a user internal state is unobservable or partially observable, the user internal state may be defined and controlled by an internal model capable of tracking and predicting the user internal state.

In an embodiment, the agent 316 may obtain a reward as a result according to a selection of an action. The reward function R(s, a, s') may be a function that defines numerical values provided when a state s is transitioned to a next state s'. In the present disclosure, a reward may be defined to correspond to an action in which the agent 316 observes a current state and determines a recommendation element. That is, in the present disclosure, because a reward is independent of a state to which an environment is transitioned, a reward function may be defined as R(s, a) which is not affected by a next state s'. The agent 316 may learn a policy of determining an action based on a reward while interacting with an environment (for example, the virtual environment 312), and therefore, the agent 316 will find better optimal actions as requests for recommendation sessions are repeated in the future.

Rewards may be designed to include negative rewards and positive rewards. For example, the negative rewards may include a case in which a user does not like a recommendation element, and the positive rewards may include a case in which a user likes a recommendation element.

As a detailed example, the negative rewards may include a case in which a user does not like a recommended movement, a case in which a user determines that a recommended movement is too easy or difficult, a case in which a recommended movement is excessively repeated, a case in which a recommended movement does not meet a user's workout style, etc. However, the negative rewards are not limited to the above-described examples.

As a detailed example, the positive rewards may include a case in which a user likes a recommended movement, a case in which a recommended movement meets a user's preferred workout difficulty level, a case in which a recommended movement improves variety of movements in a workout recommendation session, a case in which a recommended movement corresponds to focused muscles of user data, etc. However, the positive rewards are not limited to the above-described examples.

In an embodiment, the virtual environment 312 may transfer, to the agent 316, an action mask for increasing a training process speed of the agent 316 and applying limitation to a specific action. For example, the action mask may mask a specific action with 1 or 0. In this case, an action indicated as 1 may be selectable and an action indicated as 0 may not be selectable. The agent 316 may determine an action by considering only selectable actions. That is, the action mask may prevent the agent 316 from performing an unnecessary action, thereby reducing a training time and helping efficient training. For example, the action mask may limit an action of the agent 316. That is, the action mask may prevent the agent 316 from recommending a workout that the user has disliked or specified as not being able to perform.

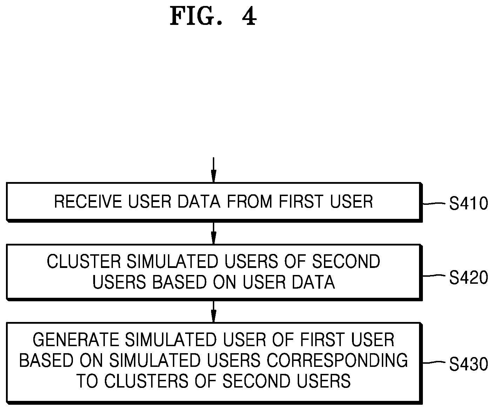

FIG. 4 is a diagram for describing an operation of, performed by a sever according to an embodiment of the present disclosure, generating a simulated user.

In operation S410, the server 2000 may receive user data from a first user. The first user may be a user who wants to receive a personalized recommendation session. For example, the first user may include a new user who has accessed the server 2000 through an electronic device (for example, a mobile phone, etc.) to use a personalized recommendation providing service. According to the first user's access to the server 2000, the server 2000 may provide a user interface (for example, a graphic user interface (GUI), a voice interface, etc.) for receiving user data. For example, the server 2000 may provide a user interface that allows a user using the service for the first time to input personal information such as age, gender, etc. Alternatively, the server 2000 may provide a user interface that allows the user to input information related to a workout which is a recommended category, such as a workout goal, a workout style, a workout duration, focused muscles, workout proficiency, a fitness level, etc. For example, the server 2000 may provide a questionnaire to obtain information related to a workout, and generate user data based on a user response to the questionnaire.

In operation S420, the server 2000 may cluster second users based on the user data.

The second users may refer to other users except for the first user, and may include, for example, existing users who use the personalized recommendation service. In this case, user data of the second users may exist. The server 2000 may obtain a cluster of second users having similar features to the first user based on the user data of the first user and the user data of the second users. For example, the server 2000 may select or extract key features from the user data of the first user and the second user and calculate similarity. In this case, various algorithms (for example, Euclidean distance measure) for measuring similarity may be used. The server 2000 may group users into clusters using a clustering algorithm (for example, hierarchical clustering, K-Means, etc.). The server 2000 may compare the user data of the first user with the clusters of the second users to identify a cluster to which the first user belongs.

In operation S430, the server 2000 may generate a simulated user of the first user based on simulated users corresponding to the clusters of the second users.

In an embodiment, because each user in the clusters of the second users has previously used the personalized recommendation providing service, simulated users corresponding to the respective second users may exist. The server 2000 may set parameters that will be included in the simulated user of the first user based on parameters included in the simulated users corresponding to the clusters of the second users. For example, the server 2000 may set parameters representing a user description p, user preferences p, etc. of the simulated user of the first user.

In an embodiment, when the server 2000 generates the simulated user of the first user, data of the second users may be insufficient. The server 2000 may identify the number of the second users, and according to the number of the second users being a predetermined value or more, the server 2000 may cluster the second users. Also, according to the number of the second users being less than the predetermined value, the server 2000 may randomly set parameters that will be included in the simulated user of the first user based on the user data of the first user.

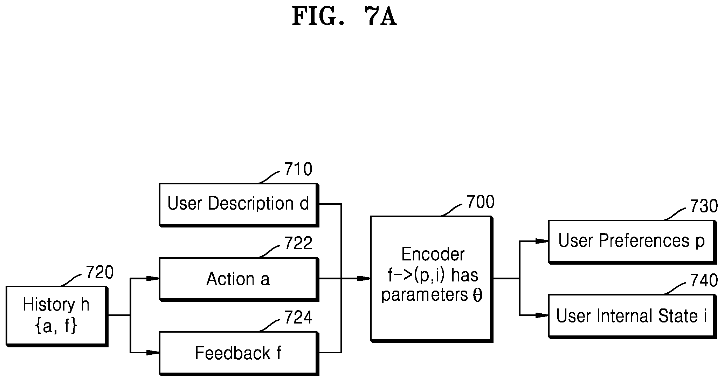

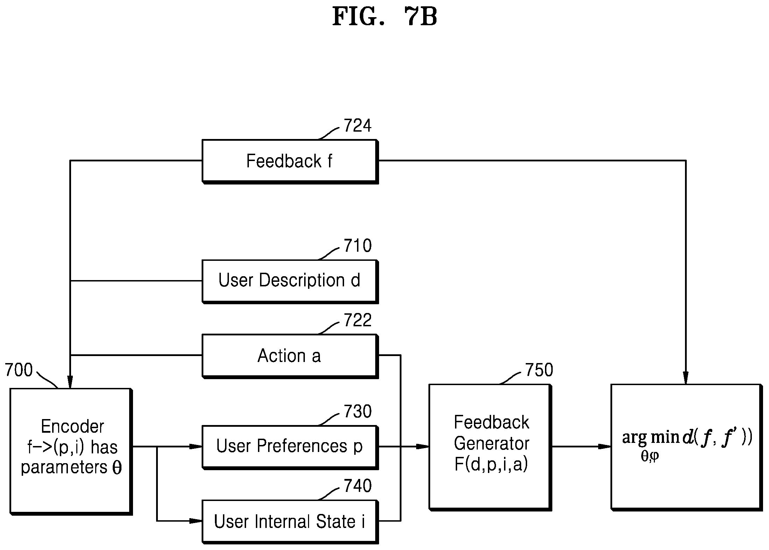

An operation of the simulated user generated by the server 2000 through the user simulator will be described in more detail with reference to FIGS. 7A and 7B.

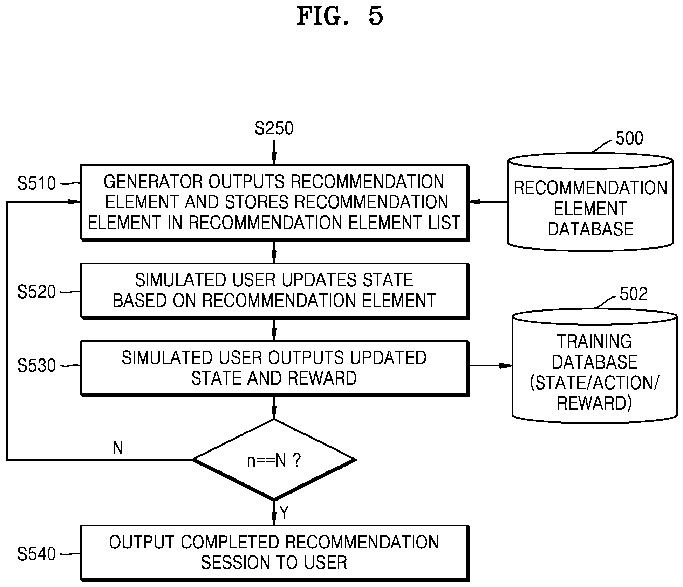

FIG. 5 is a diagram for describing an operation of, performed by a sever according to an embodiment of the present disclosure, generating a personalized recommendation session.

Referring to FIG. 5, operations S510 to S530 may correspond to operation S260 of FIG. 2. Also, in view of reinforcement learning, a generator may correspond to an agent, and a simulated user may correspond to an environment. Also, operations shown in FIG. 5 may be performed by the server 2000. For example, the server 2000 may perform data processing by using a user simulator of a reinforcement learning model or a generator.

In operation S510, the server 2000 may output a recommendation element. Determining a recommendation element may correspond to an action of an agent. That is, a generator which is an agent of a reinforcement learning model may output a recommendation element, and the output recommendation element may be stored in a list.

The server 2000 may select one from among recommendation element candidates stored in recommendation element database 500. The generator may observe a simulated user and perform an action of selecting a recommendation element based on a state of the simulated user. In this case, the recommendation element may include various attributes. For example, in a workout recommendation scenario, a movement which is a recommendation element may include, but is not limited to, attributes of a basic movement, a difficulty level, focused muscles, cardio/non-cardio, etc.

The basic movement may be a basic motion that makes a specific type of workout. A workout may be performed in various ways through different variations or auxiliary motions of the basic movement. For example, the basic movement may be squats, and in this case, the squats may include parallel squats, full squats, half squats, etc.

The difficulty level may have a predetermined range of values. A greater difficulty level value of a movement may indicate a higher difficulty level of the movement. For example, difficulty levels may be defined as values of 1 to 4, and a difficulty level of parallel squats may be defined as 1.4.

The focused muscles may represent muscles involved in performing a movement. For example, in the case of squats, the focused muscles may include quadriceps, hamstrings, etc.

The cardio/non-cardio may include whether the movement is a cardio workout to improve heart and lung functions or a non-cardio workout to strengthen muscles.

The server 2000 may output a recommendation element determined based on the state of the simulated user and the attributes of the recommendation element, by using the reinforcement learning model. That is, an action of selecting a recommendation element may be performed by the generator of the reinforcement learning model.

In operation S520, the simulated user may update a current state to a next state based on the recommendation element. That is, the simulated user may be transitioned to a next state based on a state transition function T(s, a, s'). For example, according to the simulated user receiving a movement recommendation, dynamic states of the simulated user, such as a user internal state i and a recommendation history h, may be updated.

In operation S530, the simulated user may output an updated state and a reward. The updated state and the reward may be transferred to the agent to allow the agent to determine a next action. For example, the updated state of the simulated user and the reward may be used by the agent to determine another movement for the user to perform after performing a previously recommended movement. The server 2000 may store the simulated user's previous and next states, the action, and the reward in training database 502. Data stored in the training database 502 may be used to retrain the reinforcement learning model.

In an embodiment, the server 2000 may repeat operations S510 to S530 a predetermined number of times. For example, the server 2000 may repeat operations S510 to S530 N times which is the predetermined number of times. In this case, N recommendation elements may be stored in a recommendation element list. When the server 2000 repeats operations S510 to S530 to output N recommendation elements, it can be referred to as one episode being performed.

In operation S540, the server 2000 may output a completed recommendation session to the user. For example, the server 2000 may output a workout session including N movements to the user. In this case, the user may receive the workout session from the server 2000 and sequentially perform the movements in the workout session. In an embodiment, when the server 2000 provides a recommendation session, the server 2000 may provide information related to the recommendation session to the user. For example, in the case in which the server 2000 provides a workout recommendation session, the server 2000 may provide information, such as images, video, and audio, to a user such that the user follows each of movements included in the workout session. The information related to the recommendation session, provided by the server 2000, may be output on the user's electronic device (for example, a mobile phone).

In an embodiment, the server 2000 may repeat operation S540 of generating a recommendation session M times which is a predetermined number of times. The server 2000 may repeatedly generate a recommendation session to generate a recommendation session group configured with M recommendation sessions. For example, the server 2000 may repeatedly generate a workout recommendation session to generate a workout recommendation session group configured with M recommendation sessions.

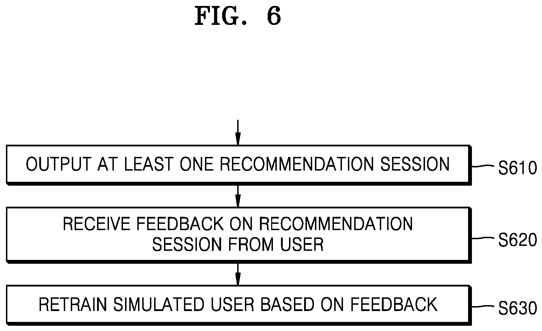

FIG. 6 is a diagram for describing an operation of, performed by a server 2000 according to an embodiment of the present disclosure, re-training a simulated user.

In operation S610, the server 2000 may output at least one recommendation session. Operation S610 may correspond to operation S270 of FIG. 2 and operation S540 of FIG. 5. For example, the server 2000 may generate a recommendation session configured with a plurality of recommendation elements and output the recommendation session, or may repeat an operation of generating a recommendation session and output a plurality of recommendation sessions. More specifically, the server 2000 may generate a workout recommendation session group configured with a workout recommendation session 1, a workout recommendation session 2, ..., a workout recommendation session M. Operations in which the server 2000 generates at least one recommendation session have been described above, and therefore, repetitive descriptions thereof will be omitted.

In operation S620, the server 2000 may receive feedback on the recommendation session from a user. For example, while the server 2000 provides the recommendation session, the server 2000 may receive feedback from the user. More specifically, while the user receives the workout recommendation session and executes each of movements according to the workout recommendation session, the user may input immediate feedback. Alternatively, after the user receives the workout recommendation session and executes all of the movements according to the workout recommendation session, the user may input feedback on the workout recommendation session.

In an embodiment, various types of feedback may be defined. Examples of feedback on a recommended workout session may include, but are not limited to, completing a movement, skipping a movement, making a movement more difficult or easier, a like/dislike response to a movement, score evaluation on an intensity of the workout session, a score for satisfaction with the workout session, etc.

In operation S630, the server 2000 may retrain a simulated user based on the feedback from the user.

In an embodiment, a reinforcement learning model may include a user simulator which is pretrained generative artificial intelligence. The user simulator may generate a simulated user that represents a virtual user corresponding to an actual user. Also, the simulated user of the user simulator may have been trained to generate simulated feedback that is similar to that from actual users. The server 2000 may perform retraining for improving precision of simulated feedback of a simulated user, based on feedback from an actual user, obtained in operation S620.

In an embodiment, the server 2000 may repeatedly generate a personalized recommendation session to generate a recommendation session group configured with a plurality of personalized recommendation sessions. In this case, whenever each of the plurality of recommendation sessions is generated, the server 2000 may retrain the simulated user. That is, after the server 2000 provides a recommendation session, the server 2000 may provide a next recommendation session by using the retrained user simulator.

In an embodiment, the reinforcement learning model may be trained by using simulated feedback output from the simulated user. While the reinforcement learning model provides a recommendation session to the simulated user and interacts with the simulated user, the reinforcement learning model may collect a recommendation history h configured with actions and feedback on the actions. More specifically, the reinforcement learning model may provide a workout recommendation session to the simulated user, and collect a recommendation history h configured with movements included in the workout recommendation session and feedback on the movements. The recommendation history may be one of segments included in a state, and the simulated feedback may be used to train an agent to select an optimal action in each state while the reinforcement learning model interacts with the simulated user.

The simulated user and the simulated feedback will be described in more detail with reference to FIGS. 7A and 7B.

FIG. 7A is a diagram for describing an operation of a user simulator according to an embodiment of the present disclosure.

In an embodiment, the server 2000 may train the user simulator such that a simulated user generated by the user simulator outputs simulated feedback f' that is similar to feedback f from an actual user. The server 2000 may adjust hyper parameters of the user simulator to improve an operation of the user simulator. The hyper parameters may be variables used to control an operation of the user simulator, and by adjusting the hyper parameters, the simulated feedback f' of the user simulator may become similar to the feedback f from the actual user.

In an embodiment, states may be classified into observable states and unobservable (or partially observable) states. For example, in a workout recommendation scenario, a user description d (710) and a recommendation history h (720) may be classified into observable states, and user preferences p (730) and a user internal state i (740) may be classified into unobservable states. The recommendation history h (720) may include an action a (722) and feedback f (724). For example, recommendation history h = [(a1, f1), (a2, f2), ..., (an, fn)].

The observable states may be directly observable and known states and the unobservable states may require inference. The server 2000 may infer an unobservable state by using the user simulator, and generate the simulated feedback f' based on the observable states d and h and the unobservable states p and i.

In an embodiment, the user simulator may include an encoder 700 for inferring an unobservable state. The encoder 700 may be a neural network model that receives the user description d (710), the action a (722), and the feedback f (724) as inputs and outputs the user preferences p (730) and the user internal state i (740). Because the user internal state i (740) is determined depending on a previous state, the encoder 700 may be implemented as a Long Short Term Memory (LSTM) model for processing time-series information. However, an implementation method of the encoder 700 is not limited thereto.

An operation of, performed by the server 2000, training the user simulator to output simulated feedback will be further described with reference to FIG. 7B.

FIG. 7B is a diagram for describing an operation of a user simulator according to an embodiment of the present disclosure.

In an embodiment, encoding may be a process for obtaining mapping from the feedback f (724) to an unobservable state (that is, (p, i) = Encode (f, o, a)). The server 2000 may use the user simulator to infer an unobservable state by encoding an observable state included in data of actual users. That is, the user simulator may use the encoder 700 to encode the user description d (710), the action a (722), and the feedback f (724) which are observable states, thereby inferring the user preferences p (73) and the user internal state i (740) which are unobservable states. The encoder 700 may include a plurality of parameters capable of being adjusted through training.

In an embodiment, decoding may be a process for generating simulated feedback based on an observable state and an unobservable state (that is, f' = Decode(z, d, a)). The server 2000 may use the user simulator to decode an observable state and an unobservable state, thereby generating simulated feedback representing feedback from a simulated user. That is, the user simulator may use feedback generator 750 to decode the user description d (710), the action a (722), the user preferences p (730), and the user internal state i (740), thereby generating simulated feedback. Here, the feedback generator 750 may correspond to a decoder. The feedback generator 750 may include a plurality of parameters capable of being adjusted through training.

The server 2000 may generate simulated feedback f' and compare the simulated feedback f' to the feedback f (724) from the user. The server 2000 may cause the simulated user to output feedback that is similar to that from the actual user by minimizing a difference between the simulated feedback f' and the feedback f (724) from the user. That is, the user simulator may be trained to find a hyper parameter for minimizing the difference between the simulated feedback f' and the feedback f (724) from the user.

In an embodiment, the server 2000 may obtain a data set Dᵣ configured with data of actual users. The data set Dᵣ may include a recommendation history (action a (722) and feedback f (724)) generated by a plurality of different policies (for example, policy A and policy B) and user information (user description d (710)) that has received a recommendation. For example, the data set Dᵣ may include a user description d and a recommendation history h = [(a1, f1), (a2, f2), ..., (an, fn)] corresponding to each of a plurality of users.

The server 2000 may train the user simulator with respect to the plurality of users included in the data set Dᵣ by using data corresponding to each user. That is, the server 2000 may perform encoding and decoding on data of each user in the data set Dᵣ to tune the hyper parameter of the user simulator. This may be to reflect each user's unique state characteristics because users have different characteristics in view of behavior patterns, preferences, etc. For example, the server 2000 may train the user simulator by using data corresponding to a user A among the plurality of users.

The server 2000 may train the user simulator by using a plurality of data sets configured with different users. For example, the server 2000 may train the user simulator such that characteristics of various user groups are reflected by using a data set Dᵣ₁ configured with a first group of users and a data set Dᵣ₂ configured with a second group of users.

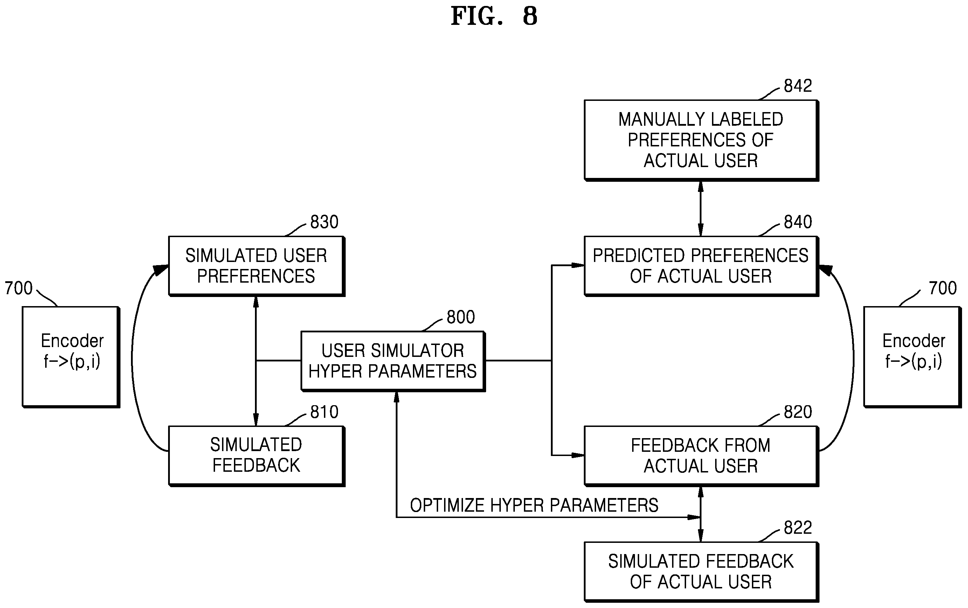

FIG. 8 is a diagram for describing an operation of, performed by a server according to an embodiment of the present disclosure, training a user simulator.

In an embodiment, the user simulator of the server 2000 may generate a simulated user. Also, the user simulator may mimic an actual user and track and manage the user's overall behaviors. For example, whether the user likes or dislikes a specific workout category, how a workout intensity affects the user's satisfaction, etc., may be tracked and a speed at which a workout intensity changes according to the user's fitness level may be adjusted. The user simulator may include hyper parameters 800 and perform the above-described operations by using the hyper parameters 800.

The server 2000 may tune values of the hyper parameters 800 to enable the user simulator to mimic the actual user's behavior, thereby optimizing the hyper parameters 800. For example, in order to make simulated feedback 810 from a simulated user similar to feedback 820 from the actual user, the server 2000 may adjust at least a part of the hyper parameters 800 to make the simulated user's preferences 830 similar to the actual user's preferences.

In an embodiment, the server 2000 may train a policy of a reinforcement learning model by using a user simulator in which initial values of the hyper parameters 800 are set. Hereinafter, a process of training the reinforcement learning model and the user simulator will be described.

The server 2000 may provide a recommendation element to an actual user based on a policy of the reinforcement learning model, and accumulate the actual user's data in an actual user data set Dᵣ. Also, the server 2000 may provide a recommendation element to a simulated user based on the policy of the reinforcement learning model, and accumulate the simulated user's data in a simulated user data set Dₛ. Each of the actual user data set Dᵣ and the simulated user data set Dₛ may include a user description and a recommendation history (actions and feedback).

The server 2000 may train the encoder 700 and a decoder (a feedback generator, not shown) by using the simulated user data set Dₛ. The encoder 700 may be trained to infer an unobservable state (including the simulated user's preferences 830), and the decoder may be trained to generate simulated feedback.

The server 2000 may generate simulated feedback 822 of the actual user by using the trained encoder 700 and the trained decoder. The server 2000 may perform optimization of the hyper parameters 800 to minimize a difference between the simulated feedback 822 and the feedback 820 from the actual user. As a result of adjusting the hyper parameters 800, the encoder 700 may infer preferences that are close to manually labeled preferences 842 of the actual user when inferring predicted preferences 840 of the actual user, and the decoder may generate feedback that is close to the feedback 820 from the actual user when generating the simulated feedback 822 of the actual user.

In an embodiment, the server 2000 may gradually train the user simulator. For example, as the number of users increases, the number of pieces of user data may also increase. Whenever the number of users increases by a predetermined criterion, the server 2000 may repeatedly perform the above-described operation of training the user simulator. For example, the server 2000 may train the user simulator by using a data set Dᵣ₁ configured with a plurality of users, and based on the number of pieces of user data increasing according to elapse of time, the server 2000 may retrain the user simulator by using a data set Dᵣ₂ including increased users. Alternatively, the server 2000 may retrain the user simulator based on various conditions. For example, the server 2000 may retrain the user simulator according to elapse of a predetermined time or when new data is obtained because a user has used a recommendation providing service. However, conditions under which the server 2000 retrains the user simulator are not limited to the above-described examples.

In an embodiment, the server 2000 may use only a part of feedback included in the feedback 820 from the actual user, as training data. The server 2000 may select a part of the feedback included in the feedback 820 from the actual user, based on a predetermined criterion. For example, feedback may be classified into three types of positive feedback, negative feedback, and neutral feedback. More specifically, a case in which a user likes a movement may correspond to positive feedback, a case in which a user changes a movement to a more difficult movement may correspond to negative feedback, and a case in which a user completes a movement may correspond to neutral feedback.

Meanwhile, the neutral feedback among the plurality of feedback types may not accurately reflect a user's preferences on a recommendation element. For example, the user may provide neutral feedback on a recommendation element even in the case in which he/she has previously given positive/negative feedback on the recommendation element or in the case in which he/she extremely likes or dislikes the recommendation element. Accordingly, the server 2000 may identify a type of the feedback 820 from the actual user and exclude a specific type of feedback from the types of feedback. For example, the server 2000 may perform a process of training the user simulator by using data remaining after excluding neural feedback.

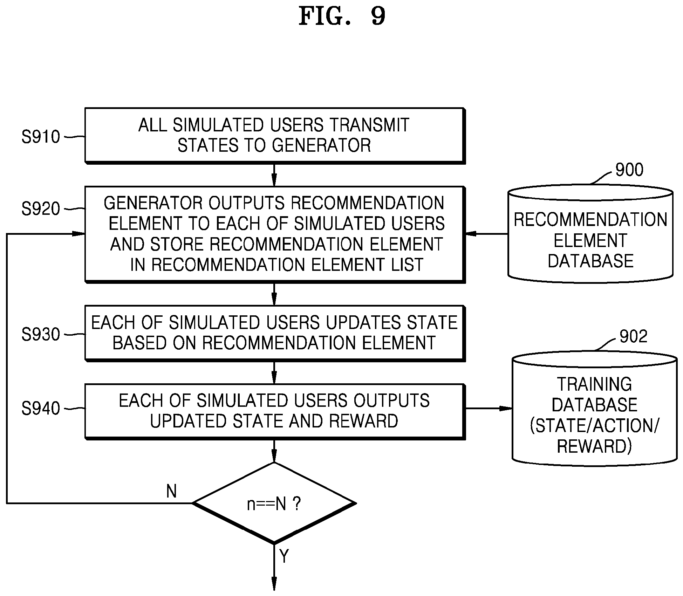

FIG. 9 is a diagram for describing an operation of, performed by a server according to an embodiment of the present disclosure, generating data for training a reinforcement learning model.

Referring to FIG. 9, operations shown in FIG. 9 may be performed by the server 2000. For example, the server 2000 may perform data processing by using the user simulator of the reinforcement learning model or the generator.

In operation S910, all simulated users may transmit states to the generator (agent). In an embodiment, a plurality of simulated users may have been generated by the user simulator. In this case, the server 2000 may request all the simulated users (or, some of all the simulated users) to operate.. In response to the request for operating all the simulated users from the server 2000, each of all the simulated users may transmit a current state to the generator in order to receive a recommendation session. For example, in a workout recommendation scenario, a state including at least one of a user description d, user preferences p, a user internal state i, or a recommendation history h may be transferred to the generator.

In operation S920, the generator may output a recommendation element to each of the simulated users and store the recommendation element in a recommendation element list. The generator may observe a state of each of the simulated users and perform an action of selecting a recommendation element based on the state of each of the simulated users. The generator may select one from among recommendation element candidates stored in recommendation element database 900 and provide the selected recommendation element to each simulated user. The same recommendation element or different recommendation elements may be provided to the respective simulated users.

In operation S930, each of the simulated users may update the current state based on the recommendation element. For example, according to each of the simulated users receiving a movement recommendation, dynamic states of each of the simulated users, such as a user internal state i and a recommendation history h, may be updated.

In operation S940, each of the simulated users may output an updated state and a reward. The update state and reward may be transferred to the generator to enable the generator to determine a next action. For example, the updated state and reward of each of the simulated users may be used by the generator to determine another movement which the user will perform after performing a previously recommended movement. The server 2000 may store previous states, next states, actions, and rewards of the simulated users in training database 902. Data stored in the training database 902 may be used as data for training the reinforcement learning model.

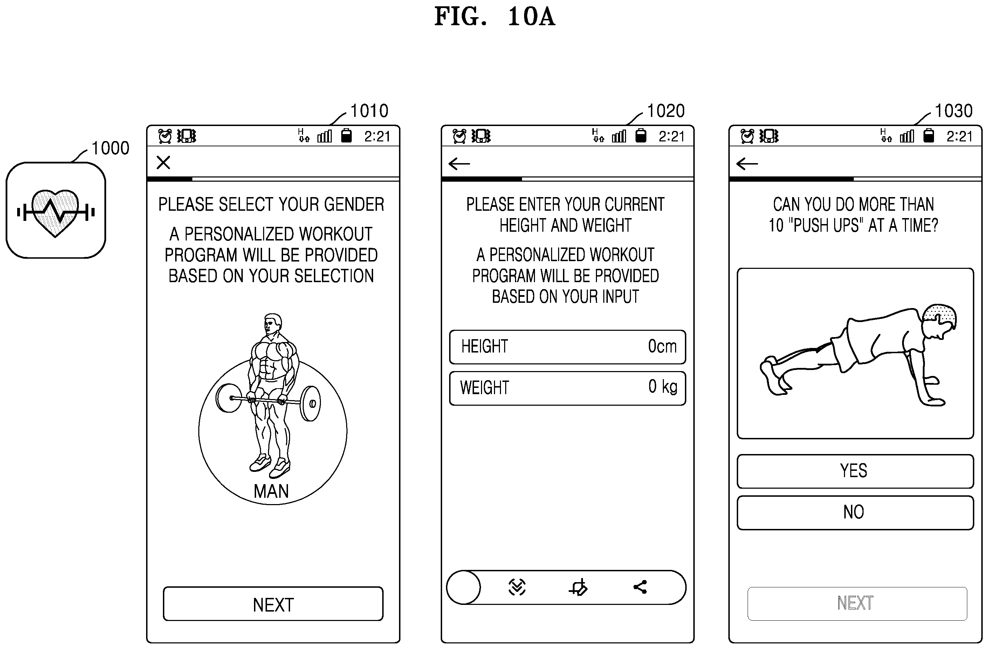

FIG. 10A is a diagram for describing an operation of, performed by a server according to an embodiment of the present disclosure, obtaining user data to provide a personalized recommendation providing service.

In an embodiment, the server 2000 may provide the personalized recommendation providing service to a user. For example, the server 2000 may provide the personalized recommendation providing service through an application 1000, and the user may execute the application 1000 through his/her electronic device (for example, a mobile phone) to access the server 2000 and receive the service.

In an embodiment, the server 2000 may obtain user data from the user through the application 1000. The server 2000 may provide various data and functions to enable the user to input user data through the application 1000. For example, the server 2000 may provide a graphical interface with a preset template to enable the user to easily input data, and may receive a user input from the user.

In an embodiment, the application 1000 may be an application that provides a workout recommendation service. In this case, the server 2000 may obtain user data related to a workout in order to provide a workout recommendation. For example, referring to a first screen 1010 of the application 1000 for a user data input, the server 2000 may receive a user input of entering a gender through the application 1000 of the user's electronic device. Or, for example, referring to a second screen 1020 of the application 1000 for a user data input, the server 2000 may receive a user input of entering a current height and weight through the application of the user's electronic device.

In an embodiment, the server 2000 may generate a question related to a recommendation element to obtain user data. For example, the server 2000 may generate a question related to workout ability to obtain information about the user's workout ability. More specifically, referring to a third screen 1030 of the application 1000 for a user data input, the server 2000 may receive a user input of responding to a question (for example, Can you do more than 10 "Push Ups" at a time?) output through the application of the user's electronic device.

Meanwhile, the user data obtained by the server 2000 is not limited to the above-described examples. For example, according to a workout recommendation scenario, the server 2000 may obtain user data including personal information related to a user (for example, age, gender, etc.) and user input information (for example, a workout goal, a workout style, a workout duration, focused muscles, workout proficiency, a fitness level, etc.) related to a workout which is a recommended category.

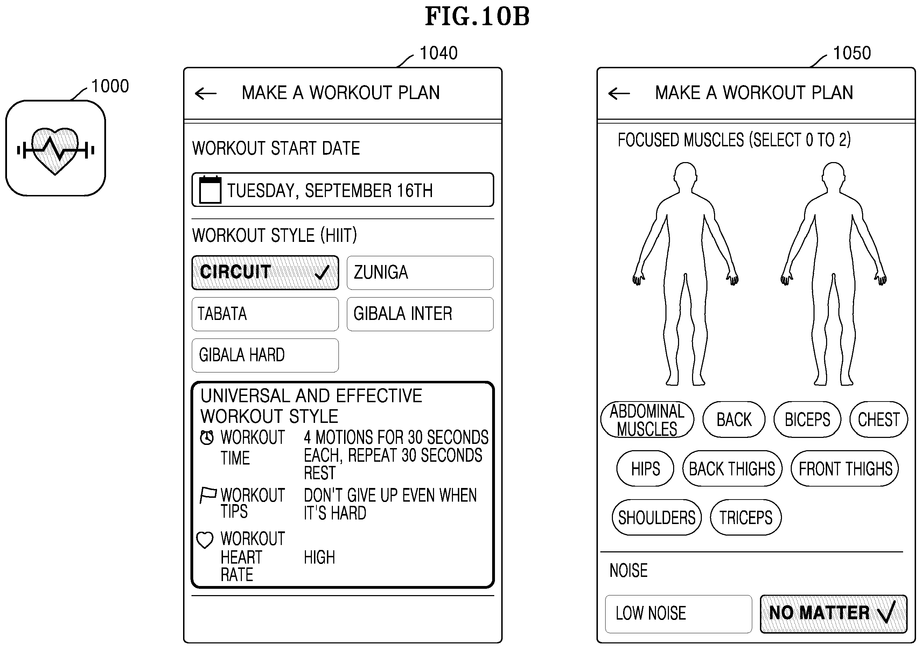

FIG. 10B is a diagram for describing an operation of, performed by a server according to an embodiment of the present disclosure, obtaining user data to provide a personalized recommendation providing service.

In an embodiment, the server 2000 may obtain user data related to a recommendation category. For example, according to a recommendation category being a workout, the server 2000 may generate a form for a user data input related to a workout to provide a workout recommendation. For example, the server 2000 may generate a data input form such as "Make a Workout Plan".

More specifically, referring to a fourth screen 1040 of the application 1000 for a user data input, the server 2000 may generate a form for making a workout plan and receive a user input of selecting a workout start date, a workout style, etc. from a user. The workout style may include, for example, High Intensity Interval Training (HIIT). Types of High-Intensity Interval Training may include, but are not limited to, circuit training, Zuniga regimen, Tabata regimen, and Gibala regimen.

Alternatively, referring to a fifth screen 1050 of the application 1000 for a user data input, the server 2000 may generate a form for making a workout plan and select a user input of selecting focused muscles from a user. Focused muscles may include, but are not limited to, abdominal muscles, back, biceps, chest, hips, back thighs, front thighs, shoulders, and triceps.

Meanwhile, recommendation categories of personalized recommendations that are provided by the server 2000 are not limited to the workout. For example, recommendations for various categories, such as a diet recommendation, a media content recommendation, etc., may be provided. The server 2000 may provide the user with a form capable of obtaining user data corresponding to each recommendation category and provide an optimized recommendation for each category.

In an embodiment, when the server 2000 receives user data, the server 2000 may generate, based on the user data, a simulated user that represents a virtual user corresponding to an actual user who receives a recommendation. The simulated user may correspond to an environment in a reinforcement learning system, and at least a part of information included in a state may be set based on the user data obtained from the user. For example, a user description d including personal information (for example, age, gender, etc.) related to the user and user input information (for example, a workout goal, a workout style, a workout duration, focused muscles, workout proficiency, a fitness level, etc.) related to a workout which is a recommendation category may be set based on the user data. Or, user preferences p including movement preferences, movement difficulty level preferences, variety preferences, heart rate preferences, feedback preferences (a probability of providing a specific type of feedback), etc. may be set based on the user data.

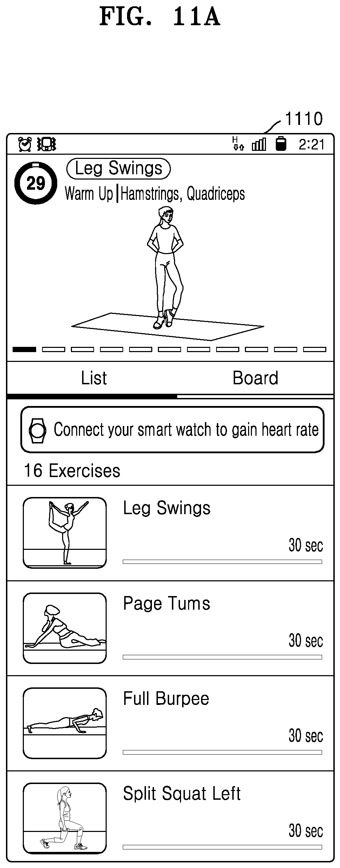

FIG. 11A is a diagram for describing an operation of, performed by a server according to an embodiment of the present disclosure, providing a personalized recommendation providing service.

In an embodiment, when the server 2000 receives a recommendation service providing request from a user, the server 2000 may generate a simulated user by using a user simulator and repeatedly determine a recommendation element through interactions between the simulated user and a recommendation generator, thereby generating a recommendation session. Operation of, performed by the server 2000, generating a recommendation session has been described above, and therefore, repetitive descriptions thereof will be omitted.

In an embodiment, the server 2000 may provide a personalized recommendation providing service through an application. In this case, the user may execute the application through his/her electronic device (for example, a mobile phone, etc.) to access the server 2000 and receive a recommendation session.

A recommendation session may include a plurality of recommendation elements. For example, a recommendation session may be configured with N predetermined recommendation elements More specifically, referring to a first screen 1110 of the application that provides a recommendation session, the server 2000 may provide the user with a workout recommendation session configured with 16 movements. The first screen 1110 may include, but is not limited to, a list of recommendation elements, recommendation element items, content (for example, videos) related to the recommendation elements, etc. More specifically, the first screen 1110 may include a list of movements, movement items, execution times, videos for following the movements, etc. Also, the first screen 1110 may include a guide for obtaining additional data from the user. More specifically, a guide such as "Connect your smartwatch to measure heart rate" may be included in the first screen 1110. In this case, the server 2000 may receive sensor data from the user's another electronic device and provide a recommendation workout session to the user by additionally using the sensor data. For example, the server 2000 may receive heart rate data from the user's smart watch and provide a recommendation workout session configured with recommendation movements to the user by using the heart rate data.

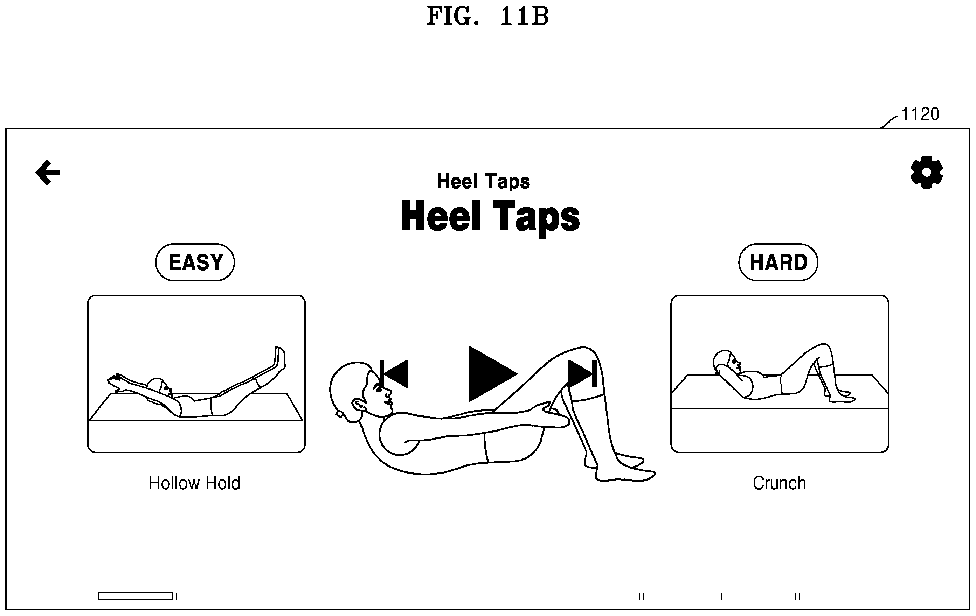

FIG. 11B is a diagram for describing an operation of, performed by a server according to an embodiment of the present disclosure, obtaining feedback on a personalized recommendation providing service.

In an embodiment, the server 2000 may obtain feedback from a user who receives a recommendation session. The server 2000 may modify at least a part of recommendation elements included in a current recommendation session based on user feedback input by the user who receives the recommendation session. Alternatively, the server 2000 may modify at least a part of recommendation elements included in a next recommendation session based on user feedback input by the user who receives a recommendation session.

In an embodiment, the user feedback may be feedback for modifying a recommendation element (or attributes of a recommendation element). For example, referring to a second screen 1120 of the application that provides a recommendation session, the server 2000 may provide, together with a currently recommended recommendation element, the user with at least one other recommendation element capable of replacing the currently recommended recommendation element. More specifically, the second screen 1120 may display "Heel Taps" which is a currently provided recommendation movement, and another recommendation movement capable of replacing "Heel Taps", such as "Hollow Hold", "Crunch", etc. Also, when the server 2000 provides an alternative recommendation element, the server 2000 may provide the user with information related to the alternative recommendation element. More specifically, the second screen 1120 may display the alternative recommendation movement "Hollow Hold" together with a difficulty level of "easy", and may display the alternative recommendation movement "Crunch" together with a difficulty level of "Hard". According to the user selecting the alternative recommendation element, the server 2000 may modify the recommendation element of the current recommendation session or the next recommendation session to the selected alternative recommendation element.

Alternatively, attributes of a recommended element may modify based on user feedback, which is not shown on the second screen 1120. For example, according to the user adjusting a difficulty level to "Hard", the server 2000 may modify a movement as in the example described above or may increase a difficulty level of the movement by modifying attributes (for example, the number of times of execution, an execution time, etc.) of the movement without modifying the movement.

In an embodiment, modifying a recommendation session by reflecting user feedback in the server 2000 may include retraining and updating the reinforcement learning model.

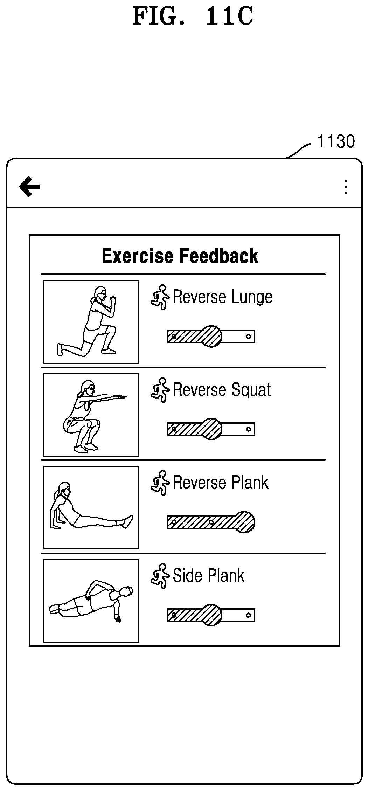

FIG. 11C is a diagram for describing an operation of, performed by a server according to an embodiment of the present disclosure, obtaining feedback on a personalized recommendation providing service.

In an embodiment, the server 2000 may obtain feedback from a user who has received a recommendation session. The server 2000 may modify at least a part of recommendation elements included in a next recommendation session based on the user feedback input received after the provision of the recommendation session.

In an embodiment, the user feedback may be feedback for modifying a recommendation element (or attributes of a recommendation element). For example, referring to a third screen 1130 of an application that provides a recommendation session, the server 2000 may provide the user with a feedback menu for recommendation elements included in the recommendation session after providing the recommendation session. The feedback menu for the recommendation elements may be configured to provide feedback on each of the recommendation elements. More specifically, on the third screen 1130, a menu capable of providing feedback on "Reverse Lunge", "Assisted Squat", "Reverse Plank", and "Side Plank", etc., which are recommendation movements included in a recently provided workout recommendation session, may be displayed. According to the user providing feedback on a recommendation element, the server 2000 may modify the recommendation element based on the feedback on the recommendation element. For example, referring to the third screen 1130, the user may provide feedback to lower a difficulty level of "Assisted Squat" and feedback to raise a difficulty level of "Reverse Plank". In this case, the server 2000 may modify recommendation elements or adjust attributes of recommendation elements by reflecting the feedback from the user when the server 2000 provides a next recommendation session.

In an embodiment, modifying a recommended session by reflecting user feedback in the server 2000 may include retraining and updating the reinforcement learning model.

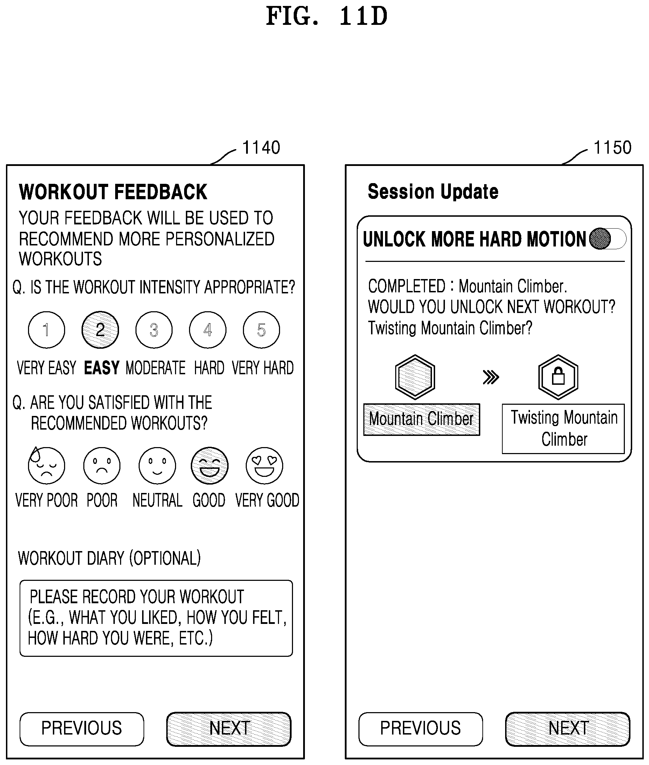

FIG. 11D is a diagram for describing an operation of, performed by a server according to an embodiment of the present disclosure, obtaining feedback on a personalized recommendation providing service.

FIG. 11D shows an example of a part of various methods of graphic interfaces which the server 2000 provides to obtain feedback from a user.

For example, referring to a fourth screen 1140 of an application that provides a recommendation session, the server 2000 may provide simple feedback menu for a recommendation session. Simple feedback may be, but is not limited to, requesting overall feedback on the recommendation session rather than requesting feedback on each of recommendation elements included in the recommendation session. More specifically, after the server 2000 provides a workout recommendation session, the server 2000 may generate a question requesting an evaluation of the workout recommendation session. For example, the server 2000 may provide questions such as "Is the workout intensity appropriate?" and "Are you satisfied with the recommended workouts?" and obtain user feedback that response to the questions. After the server 2000 obtains the user feedback, the server 2000 may modify at least a part of the recommendation session based on the feedback.

By providing the simple feedback menu, the server 2000 may enable the user to easily optimize a personalized recommendation session without hassle of individually evaluating recommendation elements in the recommendation session.

As another example, referring to a fifth screen 1150 of the application that provides a recommendation session, the server 2000 may provide a feedback menu that guides a modification of a recommendation session. Guiding a modification of a recommendation session may be, but is not limited to, guiding a modifiable recommendation element for a user and requesting feedback on whether to modify. More specifically, the server 2000 may guide unlocking of a more difficult motion. For example, the server 2000 may guide the user that a currently completed movement is "Mountain Climber" and a movement of a next difficulty level, "Twisting Mountain Climber", can be unlocked, and obtain user feedback that responds to whether to unlock. According to the server 2000 obtaining the user feedback, the server 2000 may modify at least part of the recommended session based on the feedback.

By providing a guide feedback menu, the server 2000 may enable the user to modify a recommended element based on a guide when he or she wants to modify the recommended element but does not know what to modify the recommendation element. Accordingly, the server 2000 may enable the user to easily optimize a personalized recommendation session.

In an embodiment, modifying a recommended session by reflecting user feedback in the server 2000 may include a process of retraining and updating the reinforcement learning model.

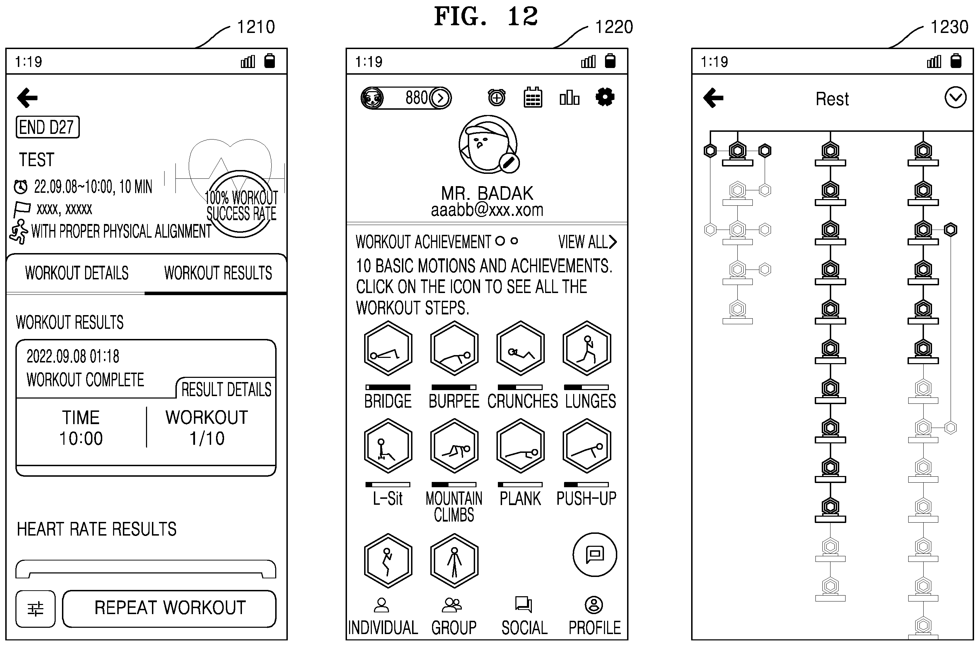

FIG. 12 is a diagram for describing an operation of, performed by a server according to an embodiment of the present disclosure, additionally providing information related to a provided recommendation session.

In an embodiment, while the server 2000 provides a personalized recommendation session to a user, the server 2000 may additionally provide information related to the personalized recommendation session to the user. The information related to the personalized recommendation session may include, but is not limited to, for example, summary information of the provided recommendation session, information about a user who receives the recommendation session, etc.

Information related to a recommendation session may depend on a category of the recommendation session. Hereinafter, a case in which a recommendation category is a workout will be described as an example.

Referring to a first screen 1210 of an application that provides a recommendation session, the first screen 1210 may include summary information of workout results. The summary information of workout results may include, but is not limited to, a workout time, a workout goal, heart rate results, and information about movements included in a workout recommendation session.

Referring to a second screen 1210 of the application that provides the recommendation session, the second screen 1220 may include information indicating workout achievement levels which are information about a user who has received the workout. The information indicating the workout achievement levels may include, but is not limited to, movements performed by the user, achievement levels of the respective movements, difficulty levels of the respective movements, etc.

Referring to a third screen 1230 of the application that provides the recommendation session, the third screen 1230 may include a movement tree indicating current workout levels which are information about the user who has received the workout. The movement tree may include, but is not limited to, movements unlocked according to the user's achievement levels and information related to the respective movements.

According to an embodiment, the server 2000 may enhance a personalization effect by additionally providing information related to a personalized recommendation session. More specifically, as shown in FIG. 12, the server 2000 may provide a workout recommendation session together with relevant information (for example, workout results, achievement levels, a movement tree suggesting a next workout, etc.) based on workout result analysis, thereby enhancing a user's experience of receiving a personalized recommendation.

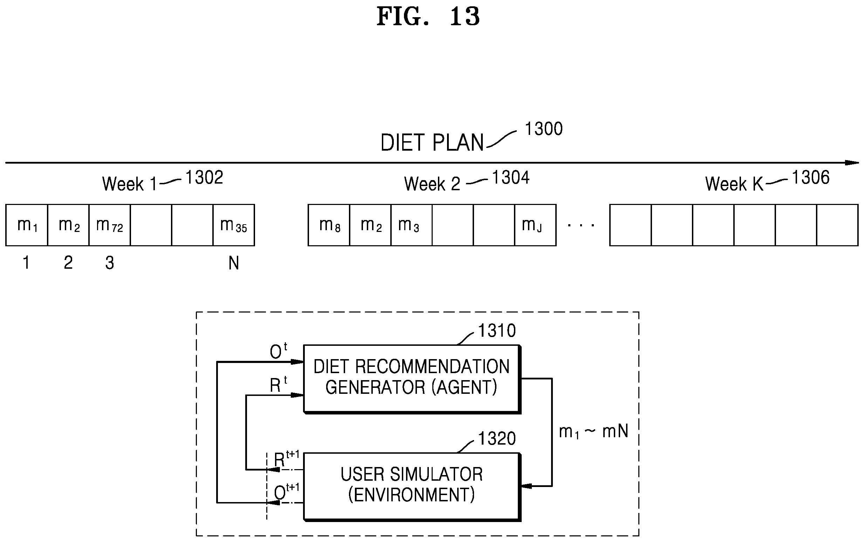

FIG. 13 is a diagram for describing an example of, performed by a server according to an embodiment of the present disclosure, providing a personalized recommendation session.

The server 2000 may provide personalized recommendation sessions for various categories. FIG. 13 shows an example of a personalized diet recommendation. However, this is only an example to explain that a technical idea of the present disclosure is applicable to various recommendation categories, and is not intended to limit recommendation categories.

In an embodiment, providing user with a personalized recommendation session for a specific recommendation category may need to satisfy several constraints. As an example of the constraints, for a diet recommendation, a weekly meal plan may need to be provided to the user, planned diets may need to be followed for several weeks, and a variety of meals may need to be planned to reflect the user's preferences, allergies, and available foods/ingredients. Additionally, variety of ingredients should not lead to food waste, and costs required to maintain the diets should be kept within a budget. The server 2000 may provide a personalized diet recommendation session to a user while satisfying various constraints by utilizing the reinforcement learning model including the user simulator.

In an embodiment, the server 2000 may provide a diet plan 1300 to the user by using the reinforcement learning model. The diet plan 1300 may include a plurality of diet recommendation sessions. For example, as shown in FIG. 13, a first week diet Week 1 (1302), a second week diet Week 2 (1304), ..., a K-th week diet Week K (1306) may be included in the diet plan 1300.

In an embodiment, the reinforcement learning model may include a diet recommendation generator 1310 that determines an action of generating a diet recommendation, and a user simulator 1320 that generates synthetic data to interact with the diet recommendation generator 1310.

In a diet recommendation scenario, the user simulator 1320 may generate a simulated user representing a virtual user who receives a diet recommendation. The simulated user may transmit a state to the diet recommendation generator 1310, and the diet recommendation generator 1310 may determine a meal which is a recommended element. The state may contain all information required to determine a reward at a given time and a transition to a subsequent state. The state may be divided into various segments (s = Concat(s1, s2, s3, ..., sn)).

In the diet recommendation scenario, the state may include at least one of a user description d, user preferences p, a user internal state i, or a recommendation history h.

The user description included in the state may include, but is not limited to, personal information about the user (for example, age, gender, etc.) and user input information (for example, food allergies, regional food ingredients restrictions, a user's budget, a user's dining place, refrigeration requirements, access to a cooking area, initial weight, blood sugar, etc.) related to a diet which is a recommendation category.

The user preferences included in the state may include, but are not limited to, food category preferences, food cooking time preferences, available ingredient preferences, recipe complexity, food/ingredient variety preferences, and feedback preferences (for example, a probability that the user will provide feedback of skipping/substituting a food/ingredient).

The user internal state included in the state may include elements that are affected when the user receives a recommendation. For example, the user internal state may include, but is not limited to, the user's weight, blood sugar, disliked ingredients, or a probability that the user will skip an ingredient when a complex recipe is recommended.

The recommendation history included in the state may include a history of recommended elements recommended to the user and feedback on the recommended elements. For example, food and feedback on food, which are recommended elements included in a diet recommendation session, may be included in the recommendation history.

Meanwhile, the user internal state and recommendation history included in the state may be classified into dynamic. In a state transition function, dynamic segments may be defined to change according to a transition to a next state. In this case, the dynamic segments may be classified into "deterministic (p=1)" and "non-deterministic (p≠1)". For example, a recommendation history which is a dynamic segment may be classified into deterministic because the recommendation history results from newly adding a recommendation element determined by a current action to an end of a list of recommendation sessions. Also, user feedback, such as like/dislike for a recommended element, may be classified into non-deterministic because the user feedback is defined by a probability. Likewise, a state transition of a user internal state may also be classified into non-deterministic. Because a user internal state is unobservable or partially observable, the user internal state may be defined and controlled by an internal model capable of tracking and predicting the user internal state.

Rewards may be designed to include negative rewards and positive rewards. For example, the negative rewards may include a case in which a user does not like a recommendation element, and the positive rewards may include a case in which a user likes a recommendation element. Rewards may be designed to reflect, for example, variety of recommended foods for meals, food ingredient costs, food nutrient diversity, like/dislike feedback on foods, and whether a weight/blood sugar goal is achieved.

Meanwhile, in addition to the components of the reinforcement learning system using the user simulator as described above, specific operations including interactions between the agent and the environment in the reinforcement learning system have been described with reference to the previous drawings, and therefore, repetitive descriptions thereof will be omitted.

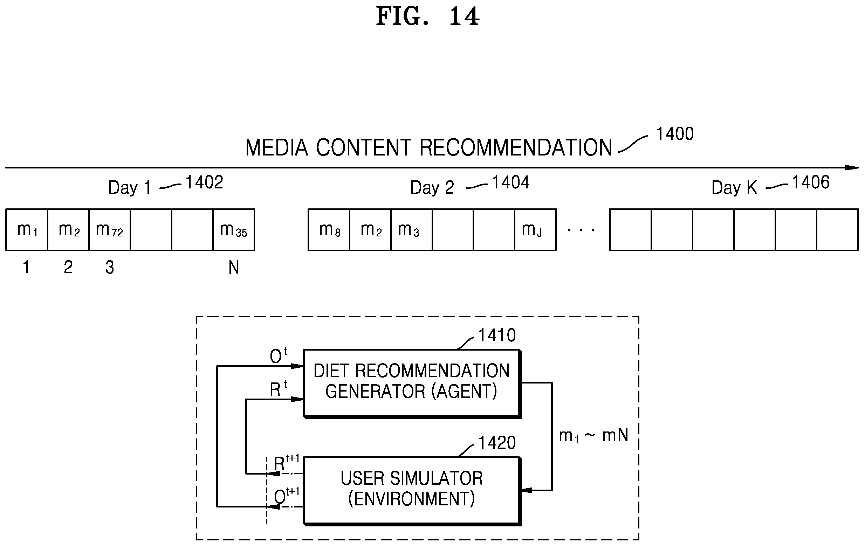

FIG. 14 is a diagram for describing an example of, performed by a server according to an embodiment of the present disclosure, providing a personalized recommendation session.

The server 2000 may provide personalized recommendation sessions for various categories. FIG. 14 shows an example of a personalized media content recommendation. However, this is only an example to explain that a technical idea of the present disclosure is applicable to various recommendation categories, and is not intended to limit recommendation categories.

In an embodiment, providing a user with a personalized recommendation session for a specific recommendation category may need to satisfy several constraints. As an example of the constraints, for a media content recommendation, today's content may need to be recommended to the user, the user may need to be engaged with a system for a long term, the user's preferences (a genre, a playback time, a viewing time zone) may need to be reflected, and a variety of content may need to be provided. In addition, a regular daily content viewing schedule may need to be provided, content provided from various content platforms (for example, a N content platform, a Y content platform, etc.) may need to be integrated and recommended, and next content may need to be recommended based on a previous content viewing history. The server 2000 may provide a personalized media content recommendation session to a user while satisfying various constraints by utilizing the reinforcement learning model including the user simulator.

In an embodiment, the server 2000 may provide a media content recommendation 1400 to a user by using the reinforcement learning model. The media content recommendation 1400 may include a plurality of media content recommendation sessions. For example, as shown in FIG. 4, first day media content Day 1 (1402), second day media content Day 2 (1404), ..., K-th day media content Day K (1406) may be included in the media content recommendation 1400.

In an embodiment, a reinforcement learning model may include a media content recommendation generator 1410 that determines an action of generating a media content recommendation, and a user simulator 1420 that generates synthetic data to interact with the media content recommendation generator 1410.

In a media content recommendation scenario, the user simulator 1320 may generate a simulated user representing a virtual user who receives a media content recommendation. The simulated user may transfer a state to the media content recommendation generator 1410 and the media content recommendation generator 1410 may determine content that is a recommendation element. The state may contain all information required to determine a reward at a given time and a transition to a subsequent state. The state may be divided into various segments (s = Concat(s1, s2, s3, ..., sn)).

In the media recommendation scenario, the state may include at least one of a user description d, user preferences p, a user internal state i, or a recommendation history h.

The user description included in the state may include, but is not limited to, personal information (for example, age, gender, a marital status, presence of children, interests, etc.) about the user and user input information (for example, a content genre, a content language, an actor, a content region, a previous residence history, etc.) related to media content which is a recommendation category.

The user preferences included in the state may include, but are not limited to, content genre preferences, actor preferences, region preferences, screening time preferences, content variety preferences, and feedback preferences (for example, a probability that the user will provide feedback of skipping/replacing content).

The user internal state included in the state may include elements that are affected when the user receives a recommendation. For example, the user internal state may include, but is not limited to, the user's tracked viewing time, a service reconnection rate, a content recommendation hit/miss ratio, a content decision time, a percentage of viewed content with respect to full content, and content evaluation.

The recommendation history included in the state may include a history of recommended elements recommended to the user and feedback on the recommended elements. For example, content and feedback on content, which are recommendation elements included in the media content recommendation session, may be included in the recommendation history.

Meanwhile, the user internal state and recommendation history included in the state may be classified into dynamic. In a state transition function, dynamic segments may be defined to change according to a transition to a next state. In this case, the dynamic segments may be classified into "deterministic (p=1)" and "non-deterministic (p≠1)". For example, a recommendation history which is a dynamic segment may be classified into deterministic because the recommendation history results from newly adding a recommendation element determined by a current action to an end of a list of recommendation sessions. Also, user feedback, such as like/dislike for a recommended element, may be classified into non-deterministic because the user feedback is defined by a probability. Likewise, a state transition of a user internal state may also be classified into non-deterministic. Because a user internal state is unobservable or partially observable, the user internal state may be defined and controlled by an internal model capable of tracking and predicting the user internal state.

Rewards may be designed to include negative rewards and positive rewards. For example, the negative rewards may include a case in which a user does not like a recommendation element, and the positive rewards may include a case in which a user likes a recommendation element. Rewards may be designed to reflect, for example, variety of content provided in a day, variety of content provided across days, the number of pieces of viewed content, like/dislike feedback on content, and whether content is selected for a recommendation.

Meanwhile, in addition to the components of the reinforcement learning system using the user simulator as described above, specific operations including interactions between the agent and the environment in the reinforcement learning system have been described with reference to the previous drawings, and therefore, repetitive descriptions thereof will be omitted.

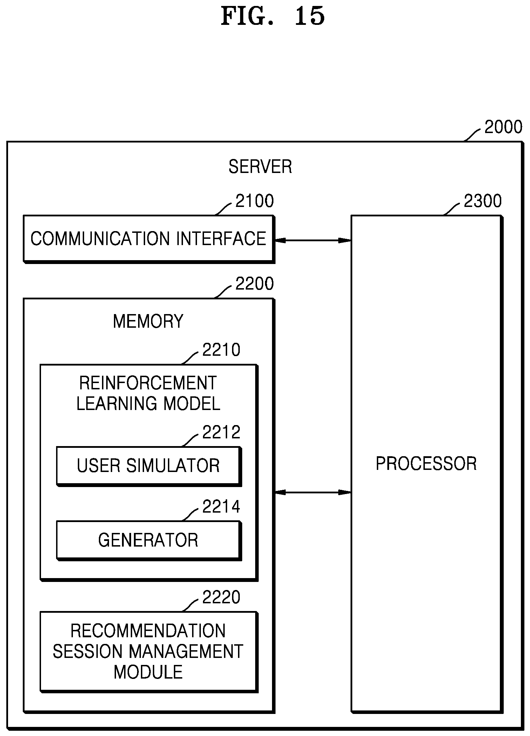

FIG. 15 is a block diagram showing a configuration of a server according to an embodiment of the present disclosure.

In an embodiment, the server 2000 may include a communication interface 2100, memory 2200, and a processor 2300.

The communication interface 2100 may perform data communication with other electronic devices under a control by the processor 2300.

The communication interface 2100 may include a communication circuit capable of performing data communication between the server 2000 and other devices through at least one of data communication methods including, for example, wireless local area network (LAN), wireless-fidelity (Wi-Fi), Bluetooth, zigbee, Wi-Fi Direct (WFD), infrared communication (infrared Data Association (IrDA)), Bluetooth Low Energy (BLE), Near Field Communication (NFC), Wireless Broadband Internet (Wibro), World Interoperability for Microwave Access (WiMAX), Shared Wireless Access Protocol (SWAP), Wireless Gigabit Alliance (WiGig), and radio frequency (RF) communication.

The communication interface 2100 may transmit/receive data for providing a personalized recommendation service to/from an external device. For example, the communication interface 2100 may receive user data from a user's electronic device (for example, a mobile phone, etc.), transmit a personalized recommendation session to the user's electronic device 2000, and receive user feedback from the user's electronic device.

Instructions, data structures, and program codes which are readable by the processor 2300 may be stored in the memory 2200. Operations that are performed by the processor 2300 may be implemented by executing instructions or codes of a program stored in the memory 2200.

The memory 2200 may include a flash memory type, a hard disk type, a multimedia card micro type, and card type memory (for example, Secure Digital (SD) memory or eXtreme Digital (XD) memory), and may include non-volatile memory including at least one of Read-Only Memory (ROM), Electrically Erasable Programmable Read-Only Memory (EEPROM), Programmable Read-Only Memory (PROM), magnetic memory, a magnetic disk, or an optical disk, and volatile memory, such as Random Access Memory (RAM) or Static Random Access Memory (SRAM).

The memory 2200 may store one or more instructions and/or programs to operate the server 2000 to provide a personalized recommendation session service. For example, a reinforcement learning model 2210 and a recommendation session management module 2220 may be stored in the memory 2200. The reinforcement learning model 2210 may include a user simulator 2212 and a generator 2214.

The processor 2300 may include overall operations of the server 2000. For example, the processor 2300 may execute one or more instructions of a program stored in the memory 2200 to control overall operations of the server 2000 to generate a personalized recommendation session. One or more processors 2300 may be provided.

The processor 2300 may be configured with at least one of, for example, a Central Processing Unit, a microprocessor, a Graphic Processing Unit, an Application Specific Integrated Circuits (ASICs), Digital Signal Processors (DSPs), Digital Signal Processing Devices (DSPDs), Programmable Logic Devices (PLDs), a Field Programmable Gate Arrays (FPGAs), an Application Processor, a Neural Processing Unit, or an Al-specific processor designed with a hardware structure specialized for processing an Al model, but is not limited thereto.

In an embodiment, the processor 2300 may use the reinforcement learning model 2210 to generate a personalized recommendation session. The reinforcement learning model 2210 may include a user simulator 2212 and a generator 2214. The user simulator 2212 may generate a simulated user that represents a virtual user corresponding to an actual user who receives a recommendation. The generator 2214 may generate a recommendation element included in a recommendation session that is provided to a user. In a reinforcement learning system according to the present disclosure, an actual user (or a recommendation providing application) who receives a recommendation may correspond to an environment. Also, the generator 2214 that determines and provides a recommended element may correspond to an agent. In this case, a simulated user may be referred to as a virtual environment because the simulated user simulates an actual user. The simulated user may be used to track the actual user's internal state and manage a history of recommendations provided to the actual user. Also, the simulated user may generate simulated feedback that is used to train the reinforcement learning model. In this case, the user simulator 2212 may have been trained to generate simulated feedback that is similar to those from actual users based on feedback data from the actual users. The personalized recommendation session may be generated through an interaction between the simulated user generated by the user simulator 2212 and the generator 2212.

The processor 2300 may provide a personalized recommendation session (or a recommendation session group) to the user by using the reinforcement learning model 2210. The processor 2300 may train the reinforcement learning model 2210 by using feedback from a user and/or synthetic data generated by the user simulator 2212.

In an embodiment, the processor 2300 may use the recommendation session management module 2220 to manage a recommendation session that is provided to the user. For example, the processor 2300 may use the recommendation session management module 2220 to manage a history of recommendation sessions provided to the user, generate and manage information (for example, results of providing recommendation sessions, summary information, information about the user who receives the recommendation sessions, etc.) related to the recommendation sessions, and provide the information to the user.

Descriptions about operations of the reinforcement learning model 2210 and the recommendation session management module 2220 are included in those given with reference to the previous drawings, and therefore, repetitive descriptions will be omitted.

Meanwhile, the module and model stored in the above-described memory 2200 are for convenience of description and are not necessarily limited. Another module may be added to implement the above-described embodiments, and some modules of the above-described modules may be implemented as one module.

In the case in which the method according to an embodiment of the present disclosure includes a plurality of operations, the plurality of operations may be performed by a single processor or a plurality of processors. For example, in the case in which a first operation, a second operation, and a third operation are performed by the method according to an embodiment, all of the first operation, the second operation, and the third operation may be performed by a first processor, or the first operation and the second operation may be performed by the first processor (for example, a general-purpose processor) while the third operation may be performed by the second processor (for example, an Al-specific processor). An Al-specific processor which is an example of the second processor may perform operations for training/inferring an Al model. However, embodiments of the present disclosure are not limited thereto.

One or more processors according to the present disclosure may be implemented as a single-core processor or a multi-core processor.

In the case in which the method according to an embodiment of the present disclosure includes a plurality of operations, the plurality of operations may be performed by a single core or by a plurality of cores included in the one or more processors.

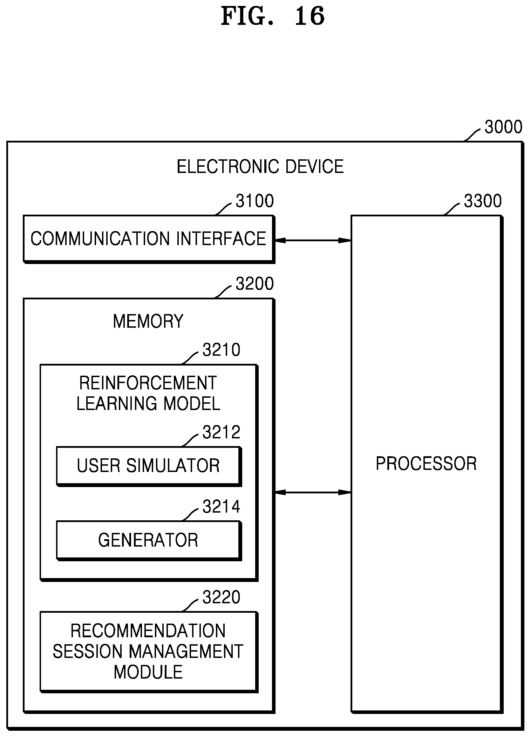

FIG. 16 is a block diagram showing a configuration of an electronic device according to an embodiment of the present disclosure.

In an embodiment, operations of the above-described server 2000 may be performed by an electronic device 3000. The electronic device 3000 may be, for example, a user's electronic device and may be implemented as various types of computing devices capable of operating a reinforcement learning model, such as a mobile phone, a tablet, etc. That is, operations of the present disclosure may be on-device services that may be implemented by a single device. Accordingly, a user may receive a personalized recommendation session by using only his/her electronic device.

The electronic device 3000 may include a communication interface 3100, memory 3200, and a processor 3300, and a reinforcement learning model 3210 and a recommendation session management module 3220 may be stored in the memory 3200. The reinforcement learning model 3210 may include a user simulator 322 and a generator 3214.

Operations of the communication interface 3100, the memory 3200, and the processor 3300 of the electronic device 3000 may be similar to operations of the communication interface 2100, the memory 2200, and the processor 2300 of the server 2000 of FIG. 15, and therefore, repetitive descriptions thereof will be omitted.

The present disclosure relates to a method of providing a highly personalized recommendation to a user based on reinforcement learning. Also, the present disclosure relates to a method of training a high-performance reinforcement learning model only with a small amount of actual user data by using synthetic data generated by a user simulator. It should be noted that technical objects of the present disclosure are not limited to the above-described technical objects, and other technical objects not mentioned will be apparent to one of ordinary skill in the technical art to which the present disclosure belongs from the descriptions of this specification.

According to an aspect of the present disclosure, a method of, performed by a server, providing a personalized recommendation session based on reinforcement learning may be provided.

The method may include obtaining user data.

The method may include generating a simulated user representing a virtual user corresponding to an actual user who receives a recommendation, based on the user data.

The method may include determining an action based on the simulated user state.

The action may determine a recommendation element to be included in a recommendation session to be provided to the user.

The method may include updating the state of the simulated user.

The method may include identifying an updated state of the simulated user and a reward for the recommendation element output from the simulated user.

The method may include generating a personalized recommendation session by repeatedly determining the recommendation element based on the updated state of the simulated user and the reward.

The method may include outputting the personalized recommendation session.

The state of the simulated user may include at least one of a user description, a user preference, a user internal state, or a recommendation history.

The generating of the simulated user may include receiving the user data from a first user.

The generating of the simulated user may include clustering second users based on the user data.

The generating of the simulated user may include generating a simulated user of the first user based on simulated users corresponding to a cluster of the second users.

The generating of the simulated user may include identifying the number of the second users.

The generating of the simulated user may include randomly setting parameters included in the simulated user of the first user, based on the user data, based on the number of the second users being less than a predetermined value.

The method may include obtaining user feedback on the personalized recommendation session.

The method may include retraining the simulated user based on the user feedback.

The method may include generating a recommendation session group configured with a plurality of recommendation sessions by repeatedly generating the personalized recommendation session.

The retraining of the simulated user may be performed whenever each of the plurality of personalized recommendation sessions is generated.

The generating of the simulated user may include generating the simulated user by using a user simulator being pretrained generative artificial intelligence.

The user simulator may have been trained to generate simulated feedback that is similar to those from actual users, based on feedback data of the actual users.

A reinforcement learning model that provides the personalized recommendation session may have been trained in advance by using the simulated feedback.

The personalized recommendation session may be a workout recommendation session including movement recommendation elements related to a workout.

According to an aspect of the present disclosure, a server for providing a personalized recommendation session based on reinforcement learning may be provided.

The server may include: a communication interface; memory storing at least one instruction; and at least one processor configured to execute the at least one instruction stored in the memory.

The at least one processor may be configured to execute the at least one instruction to obtain user data.

The at least one processor may be configured to execute the at least one instruction to generate a simulated user representing a virtual user corresponding to an actual user who receives a recommendation, based on the user data.

The at least one processor may be configured to execute the at least one instruction to determine an action based on the simulated user state.

The action may determine a recommendation element to be included in a recommendation session to be provided to the user.

The at least one processor may be configured to execute the at least one instruction to update a state of the simulated user.

The at least one processor may be configured to execute the at least one instruction to identify an updated state of the simulated user and a reward for the recommendation element output from the simulated user.

The at least one processor may be configured to execute the at least one instruction to generate a personalized recommendation session by repeatedly determining the recommendation element based on the updated state of the simulated user and the reward.

The at least one processor may be configured to execute the at least one instruction to output the personalized recommendation session.

The state of the simulated user may include at least one of a user description, a user preference, a user internal state, or a recommendation history.

The at least one processor may be configured to further execute the at least one instruction to receive the user data from the first user.

The at least one processor may be configured to further execute the at least one instruction to cluster second users based on the user data.

The at least one processor may be configured to further execute the at least one instruction to generate a simulated user of the first user, based on simulated users corresponding to a cluster of the second users.

The at least one processor may be configured to further execute the at least one instruction to identify the number of the second users.

The at least one processor may be configured to further execute the at least one instruction to randomly set parameters included in the simulated user of the first user, based on the user data, based on the number of the second users being less than a predetermined value.

The at least one processor may be configured to further execute the at least one instruction to obtain user feedback on the personalized recommendation session.

The at least one processor may be configured to further execute the at least one instruction to retrain the simulated user based on the user feedback.

The at least one processor may be configured to further execute the at least one instruction to generate a recommendation session group configured with a plurality of recommendation sessions by repeatedly generating the personalized recommendation session.

The retraining of the simulated user may be performed whenever each of the plurality of personalized recommendation sessions is generated.

The at least one processor may be configured to further execute the at least one instruction to generate the simulated user by using a user simulator being pretrained generative artificial intelligence.

The user simulator may have been trained to generate simulated feedback that is similar to those from actual users based on feedback data from the actual users.

A reinforcement learning model that provides the personalized recommendation session may have been trained in advance by using the simulated feedback.

The personalized recommendation session may be a workout recommendation session including movement recommendation elements related to a workout.

Meanwhile, embodiments of the present disclosure may be implemented in the form of a recording medium including an instruction that is executable by a computer, such as a program module that is executed by a computer. The computer-readable medium may be an arbitrary available medium which is able to be accessed by a computer, and may include a volatile or non-volatile medium and a separable or non-separable medium. Further, the computer-readable medium may include a computer storage medium and a communication medium. The computer storage medium may include volatile and non-volatile media and separable and non-separable media implemented by an arbitrary method or technology for storing information, such as a computer readable instruction, a data structure, a program module, or other data. The communication medium may include other data of a modulated data signal, such as a computer readable instruction, a data structure, or a program module.

Also, the computer-readable storage medium may be provided in a form of non-transitory storage medium. Here, the term 'non-transitory storage medium' simply means that the storage medium is a tangible device, and does not include a signal (e.g., an electromagnetic wave), but this term does not differentiate between where data is semi-permanently stored in the storage medium and where the data is temporarily stored in the storage medium. For example, the 'non-transitory storage medium' may include a buffer in which data is temporarily stored.

According to an embodiment, a method according to various embodiments of the present disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloadable or uploadable) online via an application store or between two user devices (e.g., smart phones) directly. When distributed online, at least part of the computer program product (e.g., a downloadable app) may be temporarily generated or at least temporarily stored in the machine-readable storage medium, such as a memory of the manufacturer's server, a server of the application store, or a relay server.

The aforementioned descriptions of the present disclosure are only for illustrative purposes, and it will be apparent that those of ordinary skill in the technical field to which the present disclosure belongs can make various modifications thereto without changing the technical idea and essential features of the present disclosure. Thus, it should be understood that the embodiments described above are merely for illustrative purposes and not for limitation purposes in all aspects. For example, each component described as a single type can be implemented in a distributed type, and components described as distributed can be implemented in a combined form.

The scope of the present disclosure is shown by the claims to be described below rather than the detailed description, and it is to be construed that the meaning and scope of the claims and all modifications or modified forms derived from the equivalent concept thereof are encompassed within the scope of the present disclosure.

## Claims

1. A method of providing a personalized recommendation session based on reinforcement learning, the method comprising:
obtaining user data;
generating a simulated user representing a virtual user corresponding to an actual user who receives a recommendation, based on the user data;
determining an action based on the simulated user state, wherein the action determines a recommendation element to be included in a recommendation session to be provided to the user;
updating the state of the simulated user;
identifying an updated state of the simulated user and a reward for the recommendation element output from the simulated user;
generating a personalized recommendation session by repeatedly determining the recommendation element based on the updated state of the simulated user and the reward; and
outputting the personalized recommendation session.

2. The method of claim 1, wherein
the state of the simulated user includes
at least one of a user description, a user preference, a user internal state, or a recommendation history.

3. The method of claim 1, wherein
the generating of the simulated user comprises:
receiving the user data from a first user;
clustering second users based on the user data; and
generating a simulated user of the first user based on simulated users corresponding to a cluster of the second users.

4. The method of claim 3, wherein the generating of the simulated user comprises:
identifying the number of the second users; and
randomly setting parameters included in the simulated user of the first user, based on the user data, based on the number of the second users being less than a predetermined value.

5. The method of claim 1,
further comprising
obtaining user feedback on the personalized recommendation session; and
retraining the simulated user based on the user feedback.

6. The method of claim 5,
further comprising
generating a recommendation session group configured with a plurality of recommendation sessions by repeatedly generating the personalized recommendation session,
wherein the retraining of the simulated user is performed whenever each of the plurality of personalized recommendation sessions is generated.

7. The method of claim 1, wherein
the generating of the simulated user comprises
generating the simulated user by using a user simulator that is pretrained generative artificial intelligence.

8. A server for providing a personalized recommendation session based on reinforcement learning, the server comprising:
a communication interface;
memory storing at least one instruction; and
at least one processor configured to execute the at least one instruction stored in the memory to
obtain user data,
generate a simulated user representing a virtual user corresponding to an actual user who receives a recommendation, based on the user data,
determine an action based on the simulated user state, wherein the action determines a recommendation element to be included in a recommendation session to be provided to the user,
update a state of the simulated user,
identify an updated state of the simulated user and a reward for the recommendation element output from the simulated user,
generate a personalized recommendation session by repeatedly determining the recommendation element based on the updated state of the simulated user and the reward, and
output the personalized recommendation session.

9. The server of claim 8, wherein
the state of the simulated user includes
at least one of a user description, a user preference, a user internal state, or a recommendation history.

10. The server of claim 8, wherein
the at least one processor is configured to further execute the at least one instruction to
receive the user data from the first user,
cluster second users based on the user data, and
generate a simulated user of the first user, based on simulated users corresponding to a cluster of the second users.

11. The server of claim 10, wherein
the at least one processor is configured to further execute the at least one instruction to
identify the number of the second users,
randomly set parameters included in the simulated user of the first user, based on the user data, based on the number of the second users being less than a predetermined value.

12. The server of claim 8, wherein
the at least one processor is configured to further execute the at least one instruction to
obtain user feedback on the personalized recommendation session, and
retrain the simulated user based on the user feedback.

13. The server of claim 12, wherein
the at least one processor is configured to further execute the at least one instruction to
generate a recommendation session group configured with a plurality of recommendation sessions by repeatedly generating the personalized recommendation session, and the retraining of the simulated user is performed whenever each of the plurality of personalized recommendation sessions is generated.

14. The server of claim 8, wherein
the at least one processor is configured to further execute the at least one instruction to
generate the simulated user by using a user simulator that is pretrained generative artificial intelligence.

15. A computer-readable recording medium storing a program for executing any method of claims 1 to 7 on a computer.
